# EUROPEAN PATENT SPECIFICATION

(11) **EP 3 661 557 B1**
(45) Date of publication and mention of the grant of the patent: **03.09.2025**
(21) Application number: 18841765.3
(22) Date of filing: 31.07.2018
(51) Int. Cl.: A61K 39/00, A61K 51/00, A61P 35/02, C07K 16/28

(54) **TREATMENTS FOR A HEMATOLOGICAL MALIGNANCY**
BEHANDLUNGEN FÜR HÄMATOLOGISCHE MALIGNOME
TRAITEMENTS POUR UNE MALIGNITÉ HÉMATOLOGIQUE

(30) Priority: 31.07.2017 US 201762539114 P
(43) Date of publication of application: 10.06.2020
(60) Divisional application: 25180728.5
(73) Proprietor: Actinium Pharmaceuticals, Inc., New York, NY 10017 (US)
(72) Inventor: SETH, Sandesh, New York, NY 10128 (US); THOMAS, Keisha, Brooklyn, NY 11226 (US)
(74) Representative: Forresters IP LLP
(86) International application number: PCT/US2018/044531
(87) International publication number: WO 2019/027973

(56) References cited:
- US-A1- 2005 112 060
- US-A1- 2012 076 727
- US-A1- 2015 283 275
- KATHARINA TEILUF ET AL: "[alpha]-Radioimmunotherapy with <sup>213</sup>Bi-anti-CD38 immunoconjugates is effective in a mouse model of human multiple myeloma", ONCOTARGET, vol. 6, no. 7, 10 December 2014 (2014-12-10), pages 4692 - 4703, XP055286741, DOI: 10.18632/oncotarget.2986
- ANONYMOUS: "ASH 2017: Actinium Labeled Daratumumab Shows Enhanced Killing of Myeloma Cells over Naked Daratumumab", 30 November 2017 (2017-11-30), XP055781846, Retrieved from the Internet <URL:https://www.myelomacrowd.org/actinium-labeled-daratumumab-demonstrates-enhanced-killing-multiple-myeloma-cells-naked-daratumumab/> [retrieved on 20210303]
- BERGER MARK S ET AL: "Actinium Labeled Daratumumab Demonstrates Enhanced Killing of Multiple Myeloma Cells over Naked Daratumumab", BLOOD, AMERICAN SOCIETY OF HEMATOLOGY, US, vol. 130, no. Suppl. 1, 7 December 2017 (2017-12-07), pages 4427, XP009509383, ISSN: 0006-4971
- DAWICKI WOJCIECH ET AL: "Daratumumab- 225 Actinium conjugate demonstrates greatly enhanced antitumor activity against experimental multiple myeloma tumors", ONCOIMMUNOLOGY, vol. 8, no. 8, 3 August 2019 (2019-08-03), US, pages 1607673, XP055781847, ISSN: 2162-4011, DOI: 10.1080/2162402X.2019.1607673

## Description

### TECHNICAL FIELD

The present invention relates to compositions of a radio-labelled monoclonal antibody specific for the CD38 antigen, and more particularly, compositions comprising an alpha-emitting radionuclide labelled anti-CD38 monoclonal antibody useful for the treatment of a hematological malignancy.

### BACKGROUND

Multiple myeloma is a hematologic malignancy characterized by proliferation of a single clone of plasma cells which are normally responsible for producing antibodies. These malignant plasma cells (multiple myeloma cells) crowd out healthy blood cells in the bone marrow, circulate into the peripheral blood, and enter into new sites of the bone marrow. Thus, progression of multiple myeloma occurs through the continuous interaction between the bone marrow and the multiple myeloma cells, resulting in multiple tumors and lesions throughout the skeletal system and a median survival of 5 years. Approximately 1% of all cancers, and slightly more than 10% of all hematologic malignancies, can be attributed to multiple myeloma. The vast majority of multiple myeloma patients will relapse within a few years, and in most cases the relapsed patients do not respond to therapy.

Currently available therapies for multiple myeloma include chemotherapy, stem cell transplantation, or small molecule drugs such as immunomodulators (lenalidomide, thalidomide), proteasome inhibitors (carfilzomib, bortezomib), bisphosphonates (pamidronate, zoledronic acid). Current treatment protocols, which include a combination of chemotherapeutic agents such as vincristine, carmustine, melphalan, cyclophosphamide, adriamycin, and steroidal agents such as prednisone or dexamethasone, yield a complete remission rate of only about 5%, and median survival of approximately 36-48 months from the time of diagnosis. Recent advances using high dose chemotherapy followed by autologous bone marrow or peripheral blood mononuclear cell transplantation have increased the complete remission rate and remission duration. Yet overall survival has only been slightly prolonged, and no evidence for a cure has been obtained.

The efficacy of these available chemotherapeutic treatment regimens is limited by the low proliferation rate and development of multi-drug resistance of the multiple myeloma cells. For more than 90% of multiple myeloma patients, the disease becomes chemoresistant. As a result, alternative treatment regimens aimed at adoptive immunotherapy targeting surface antigens on plasma cells are being sought.

Multiple myeloma cells uniformly overexpress CD38, which is a 45 kD type II transmembrane glycoprotein with a long C-terminal extracellular domain and a short N-terminal cytoplasmic domain. The CD38 protein is a bifunctional ectoenzyme that can catalyze the conversion of NAD⁺ into cyclic ADP-ribose (cADPR), and cADPR into ADP-ribose, and thus regulates extracellular NAD⁺ concentrations. Moreover, cADPR has been shown to be a second messenger for intracellular Ca²⁺ mobilization. Thus, CD38 may be an essential component for regulation of intracellular Ca²⁺ flux.

CD38-therapeutic antibodies as a class, typically rely on classical Fc-dependent immune effector function as a primary mechanism of action, e.g. antibody-dependent cellular cytotoxicity (ADCC), antibody-dependent cellular phagocytosis (ADCP), and complement-dependent cytotoxicity (CDC), although recent studies suggest that targeting of immune suppressor mechanisms may also contribute to the anti-tumor activity of certain CD38 antibodies.

CD38 expression is also upregulated in a variety of other malignant hematological diseases, including but not limited to B-cell chronic lymphocytic leukemia, B-cell acute lymphocytic leukemia, Waldenström macroglobulinemia, primary systemic amyloidosis, mantle-cell lymphoma, pro-lymphocytic/myelocytic leukemia, acute myeloid leukemia, chronic myeloid leukemia, follicular lymphoma, NK-cell leukemia and plasma-cell leukemia.

Moreover, expression of CD38 has been described on epithelial/endothelial cells of different origin, including glandular epithelium in prostate, islet cells in pancreas, ductal epithelium in glands, including parotid gland, bronchial epithelial cells, cells in testis and ovary, and tumor epithelium in colorectal adenocarcinoma. Thus, diseases where CD38 expression could be involved include, but are not limited to, broncho-epithelial carcinomas of the lung, breast cancer evolving from malignant proliferation of the epithelial lining in ducts and lobules of the breast, pancreatic tumors evolving from the b-cells (e.g., insulinomas), and tumors evolving from epithelium in the gut (e.g. adenocarcinoma and squamous cell carcinoma).

Daratumumab is a first-in-class CD38-targeting antibody that has been approved in combination for the treatment of newly diagnosed multiple myeloma (MM) patients who are ineligible for autologous stem cell transplant and in relapsed or refractory MM as a single agent and in combination with standard of care. Despite the notable improvement in patient responses with anti-CD38 single agent or combination therapy, not all patients respond and management of MM remains challenging.

One obstacle potentially hindering patient responses to CD38 antibody therapy is the dependence of this approach on tumors possessing significant CD38 expression levels to elicit the above mentioned mechanisms of action. Therefore patients with low or heterogeneous tumor CD38 expression tend to poorly respond to the antibody therapy. Options to increase the therapeutic index of the drug, such as antibody-drug conjugates, where a cytotoxic toxin or chemotherapy is conjugated to the antibody, are also largely dependent on high antigen density to deliver sufficient payload to enable potent tumor cell killing. Thus, therapies that could potentially work in the setting of low CD38 expression are sought and may improve the outcomes of antibody-based therapies.

US 2015/283275 A1 discloses a radioconjugate of an anti-CD38 antibody and a radionuclide. Teiluf et al. (Oncotarget. 2015, 6(7):4692-703) relates to the preclinical development of a ²¹³Bi-conjugated anti-CD38 monoclonal antibody.

### SUMMARY

The present invention is set out in the appended set of claims. Any subject-matter falling outside the scope of the claims is provided for information or illustration purposes only. Methods of treatment of the human or animal body by therapy, referred to in this description, are not part of the present invention, but are described in relation to substances or compositions for use in such methods.

A potential solution to increase the potency and potential response to CD38-directed targeted therapies that is not dependent on high expression of CD38 is radioimmunotherapy (RIT). RIT has emerged as a useful anti-tumor therapy via the targeting of radionuclides with antibodies against tumor-associated antigens

Accordingly, the present invention relates to a pharmaceutical composition for use in accordance with appended claim 1. Particular embodiments are defined in dependent claims 2 through 10.

The present invention also relates to an article of manufacture comprising an actinium (²²⁵Ac) labelled monoclonal antibody against CD38, and a label providing dose and administration instructions, wherein the article of manufacture is as defined in appended claim 11. A particular embodiment is defined in appended claim 12.

Further disclosed herein are methods for treating a mammal having a hematological malignancy, methods for inhibiting growth and/or proliferation of a cell expressing CD38, and methods of treating a disease or disorder involving cells expressing CD38, by administration of the compositions comprising the radiolabeled monoclonal antibody against CD38.

The objects of the present invention will be realized and attained by means of the combinations specifically outlined in the appended claims. The foregoing general description and the following detailed description and examples of this invention are provided to illustrate various aspects of the present invention, and by no means are to be viewed as limiting any of the described embodiments.

### BRIEF DESCRIPTION OF THE DRAWINGS

**FIG. 1** provides a schematic diagram of a method for radiolabeling daratumumab with actinium (²²⁵Ac), wherein panel (**A**) illustrates attachment of the bifunctional chelator S-2-(4-Isothiocyanatobenzyl)-1,4,7,10 tetraazacyclododecanetetraacetic acid (p-SCN-Bn-DOTA; referred to as DOTA in the figures) to the monoclonal antibody daratumumab (DARA); and panel (**B**) illustrates radiolabeling of the DOTA-DARA conjugate with ²²⁵Ac to provide ²²⁵Ac-DARA.
**FIG. 2** provides a bar graph showing the yield for chelation of actinium (²²⁵Ac) by an immunoconjugate of the chelator DOTA and DARA, wherein the conjugation reaction is performed at 37°C or room temperature, and at various molar excesses of the DOTA chelator to DARA. DOTA-DARA at 0.3 mg/ml in 0.15M ammonium acetate buffer, pH 6.5 was labelled with ²²⁵Ac (chelation of ²²⁵Ac by DOTA) in 0.01M HCL at 1uCi/ug DARA for 60 minutes at 37°C or room temperature.
**FIG. 3A** provides a graph showing the stability on storage at 4°C of various radiolabeled DARA samples, wherein the samples are from conjugation reactions between DOTA and DARA performed at 37°C or room temperature, and at various molar excesses of DOTA to DARA.
**FIG. 3B** provides a graph showing the stability on storage at room temperature of various radiolabeled DARA samples, wherein the samples are from conjugation reactions between DOTA and DARA performed at 37°C or room temperature, and at various molar excesses of DOTA to DARA.
**FIG. 4A** provides a graph showing the binding of ²²⁵Ac- DARA conjugates to various concentrations of CD38, wherein the ²²⁵Ac- DARA conjugates are from conjugation reactions between DOTA and DARA performed at 37°C or room temperature, and at various molar excesses of DOTA to DARA.
**FIG. 4B** provides a graph showing results when various concentrations of DARA and DOTA-DARA were immobilized on plastic and incubated with the complement protein C1q. The amount of C1q bound was assessed using anti-C1q-HRP as a probe.
**FIG. 4C** provides a graph showing results when various concentrations of DARA and DOTA-DARA were added to CD38 expressing target cells. Effector cells that express luciferase when activated through FcγR(III) were added to target cells and luminescence was measured after addition of Bio-Glow^{™}. Fold induction = RLU(induced-background)/RLU(no antibody control-background). Anti-CD20 antibody, which is known to activate ADCC, was used as a positive control.
**FIGS. 5A-5D** provide graphs showing the ability of ²²⁵Ac-DARA conjugates to induce lysis of Daudi cells as compared to unlabeled DARA, ²²⁵Ac-DOTA-IgG conjugates, and untreated cells, wherein **FIGS. 5A-5D** show exposure times of 24, 48, 72, and 96 hours, respectively.
**FIG. 6** provides a summary graph of the data found in **FIGS. 5A-5D****,** indicating the percent cell lysis over time for the various concentrations of ²²⁵Ac-DARA.
**FIGS. 7A-7B** provide graphs showing the ability of various concentrations of ²²⁵Ac-DARA conjugates to induce lysis of 28BM and 28PE multiple myeloid cells, respectively, by ADCC over the course of 96 hours.
**FIGS. 8A-8C** provide bar graphs comparing cell lysis results for Daudi, 28BM, and 28PE cells on exposure to various concentration of ²²⁵Ac-DOTA-DARA conjugates, wherein **FIGS. 8A-8C** show exposure times of 48, 72, and 96 hours, respectively.
**FIG. 9** shows biodistribution of ¹¹¹In-DARA in Daudi tumor-bearing SCID mice. Mice were administered a single intraperitoneal (IP) injection of ¹¹¹In-DARA (400 µCi) and imaged with microSPECT/CT at 1, 4, 24 hrs post-injection followed by 2, 3, 7 and 10 days.

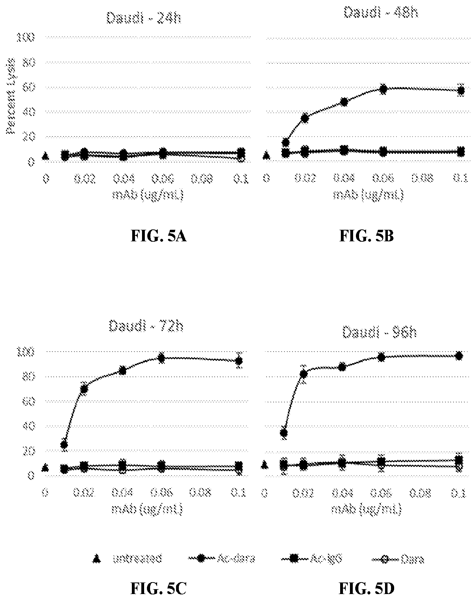

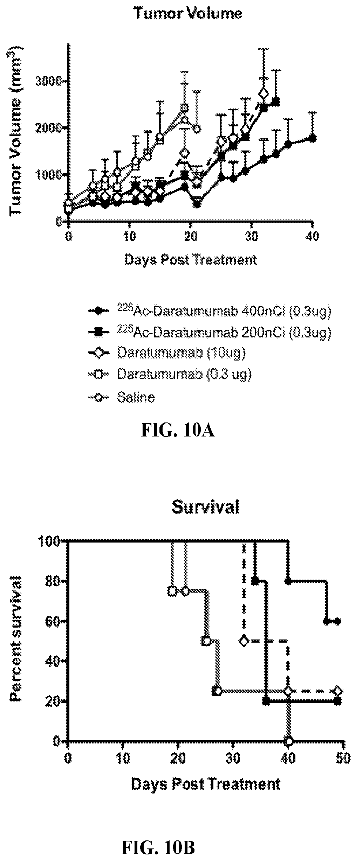

**FIGS 10A-10B** show results from radioimmunotherapy treatment (RIT) of Daudi tumor-bearing SCID mice, wherein **FIG. 5A** shown tumor volume post RIT; and **FIG. 5B** shows a Kaplan-Meier plot of mice survival post RIT. SCID mice were injected with CD38 positive Daudi cells into the right flank. When tumors reached ~200 mm³ mice were treated with a single IP injection of 200 or 400 nCi ²²⁵Ac-DARA (0.3 µg), or an equivalent dose of 0.3 µg naked DARA or a 30-fold higher dose (10 µg) naked DARA, or saline vehicle. Tumor volume was calculated using the formula V=0.5(LW²). Mice were sacrificed when the tumor volume reached 4,000 mm³.

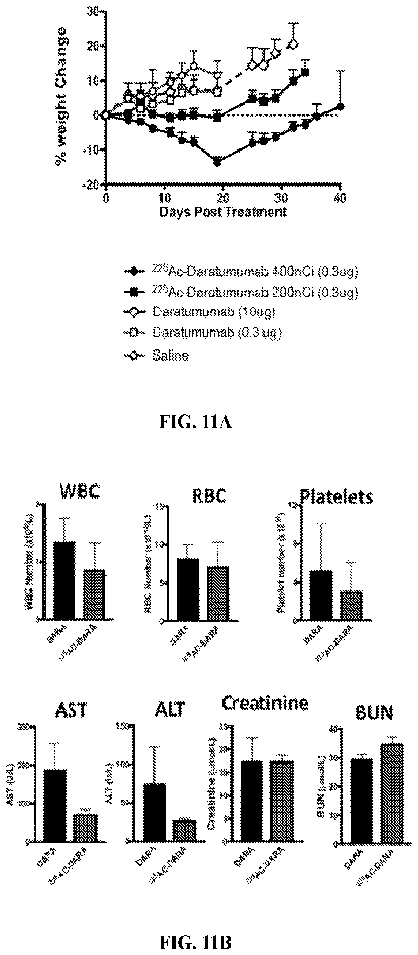

**FIGS. 11A-11B** show results from a safety evaluation of RIT with ²²⁵Ac-DARA, wherein **FIG. 11A** shows weight of the mice post RIT; and **FIG. 11B** shows hematologic, liver and kidney health parameters on day 7 post RIT. SCID mice were injected with CD38 positive Daudi cells into the right flank. When tumors reached ~200 mm³ mice were treated with a single IP injection of 200 or 400 nCi ²²⁵Ac-DARA (0.3 µg), or an equivalent dose of 0.3 µg naked DARA or a 30-fold higher dose (10 µg) naked DARA, or saline vehicle. Mice weight was monitored every 3 days post RIT. Blood was collected 7 days post RIT and analyzed for the indicated parameters.
**FIG. 12** provides the amino acid sequence of human CD38 as shown in GenBank accession number NP_001766.

### DEFINITIONS AND ABBREVIATIONS

The singular forms "a," "an," "the" and the like include plural referents unless the context clearly dictates otherwise. Thus, for example, reference to "a" diluent includes both a single diluent and a plurality of different diluents.

The term "about" when used before a numerical designation, e.g., temperature, time, amount, and concentration, including a range, indicates approximations which may vary by ±10%, ±5%, or ±1%.

"Comprising" or "comprises" is intended to mean that the compositions and methods include the recited elements, but do not exclude others. "Consisting essentially of" when used to define compositions and methods, shall mean excluding other elements of any essential significance to the combination for the stated purpose. Thus, a method consisting essentially of the elements as defined herein would not exclude other steps or composition that do not materially affect the basic and novel characteristic(s) of the claimed invention.

"Monoclonal antibody" refers to a preparation of antibody molecules of single molecular composition. A monoclonal antibody composition displays a single binding specificity and affinity for a particular epitope, or in a case of a bispecific monoclonal antibody, a dual binding specificity to two distinct epitopes. "Monoclonal antibody" therefore refers to an antibody population with single amino acid composition in each heavy and each light chain, except for possible well known alterations such as removal of C-terminal lysine from the antibody heavy chain. Monoclonal antibodies may have heterogeneous glycosylation within the antibody population. Monoclonal antibodies may be monospecific or multispecific, or monovalent, bivalent or multivalent. A bispecific antibody is included in the term monoclonal antibody.

An "epitope" refers to the target molecule site that is capable of being recognized by, and bound by, an antibody. For a protein epitope, for example, this may refer to the amino acids (and particularly their side chains) that are bound by the antibody. Overlapping epitopes include at least one to five common amino acid residues. Methods of identifying epitopes of antibodies are known to those skilled in the art and include, for example, those described in Antibodies, A Laboratory Manual, Cold Spring Harbor Laboratory, Ed Harlow and David Lane (1988).

"Humanized antibody" refers to an antibody in which the antigen binding sites are derived from non-human species and the variable region frameworks are derived from human immunoglobulin sequences. Humanized antibodies may include substitutions in the framework regions so that the framework may not be an exact copy of expressed human immunoglobulin or germline gene sequences.

"Human antibody" refers to an antibody having heavy and light chain variable regions in which both the framework and the antigen binding sites are derived from sequences of human origin. If the antibody contains a constant region, the constant region is also derived from sequences of human origin.

A human antibody comprises heavy or light chain variable regions having variable domain sequences that are "derived from" sequences of human origin wherein the variable regions of the antibody are obtained from a system that uses human germline immunoglobulin or rearranged immunoglobulin genes. Such systems include human immunoglobulin gene libraries displayed on phage, and transgenic non-human animals such as mice carrying human immunoglobulin loci. A human antibody may contain amino acid differences when compared to the human germline immunoglobulin or rearranged immunoglobulin genes due to for example naturally occurring somatic mutations or intentional introduction of substitutions in the framework or antigen binding site, or both. Typically, human antibody refers to an antibody having at least about 80%, 81%, 82%, 83%, 84%, 85%, 86%, 87%, 88%, 89%, 90%, 91%, 92%, 93%, 94%, 95%, 96%, 97%, 98%, 99% or 100% identical amino acid sequence to an amino acid sequence encoded by human germline immunoglobulin or rearranged immunoglobulin genes.

"Immunoreactivity" refers to a measure of the ability of an immunoglobulin to recognize and bind to a specific antigen.

"Isolated antibody" refers to an antibody or antibody fragment that is substantially free of other antibodies having different antigenic specificities (e.g., an isolated antibody specifically binding CD38 is substantially free of antibodies that specifically bind antigens other than human CD38). An isolated antibody that specifically binds CD38, however, may have cross-reactivity to other antigens, as indicated above. Moreover, an isolated antibody may be substantially free of other cellular material and/or chemicals. "Isolated antibody" encompasses antibodies that are isolated to a higher purity, such as antibodies that are 80%, 81%, 82%, 83%, 84%, 85%, 86%, 87%, 88%, 89%, 90%, 91%, 92%, 93%, 94%, 95%, 96%, 97%, 98%, 99% or 100% pure.

"Pharmaceutically acceptable salt" refers to acid addition salts of basic compounds, e.g., those compounds including a basic amino group, and to basic salts of acidic compounds, e.g., those compounds including a carboxyl group, and to amphoteric salts of compounds that include both an acidic and a basic moiety, such that these salts are suitable for administration in vivo, preferably to humans. Various organic and inorganic acids may be used for forming acid addition salts. Pharmaceutically acceptable salts are derived from a variety of organic and inorganic counter ions well known in the art. Pharmaceutically acceptable salts include, when the molecule contains a basic functionality, by way of example only, hydrochloride, hydrobromide, tartrate, mesylate, acetate, maleate, oxalate and the like, and when the molecule contains an acidic functionality, by way of example only, sodium, potassium, calcium, magnesium, ammonium, tetraalkylammonium, N-methylmorpholinium, and the like. In one embodiment, the pharmaceutically acceptable salt of ezatiostat is ezatiostat hydrochloride.

As used herein, "cancer" includes, without limitation, a solid cancer (e.g., a tumor) and a hematologic malignancy.

A "hematologic malignancy", also known as a blood cancer, is a cancer that originates in blood-forming tissue, such as the bone marrow or other cells of the immune system. Hematologic malignancies include, without limitation, leukemias (such as acute myeloid leukemia (AML), acute promyelocytic leukemia, acute lymphoblastic leukemia (ALL), acute mixed lineage leukemia, chronic myeloid leukemia, chronic lymphocytic leukemia (CLL), hairy cell leukemia and large granular lymphocytic leukemia), myelodysplastic syndrome (MDS), myeloproliferative disorders (polycythemia vera, essential thrombocytosis, primary myelofibrosis and chronic myeloid leukemia), lymphomas, multiple myeloma, MGUS and similar disorders, Hodgkin's lymphoma, non-Hodgkin lymphoma (NHL), primary mediastinal large B-cell lymphoma, diffuse large B-cell lymphoma, follicular lymphoma, transformed follicular lymphoma, splenic marginal zone lymphoma, lymphocytic lymphoma, T-cell lymphoma, and other B-cell malignancies.

As used herein, a subject's "peripheral blood lymphocytes" shall mean the mature lymphocytes circulating in the subject's blood. Examples of peripheral blood lymphocytes include, without limitation, peripheral blood T-cells, peripheral blood NK cells and peripheral blood B cells. A subject's peripheral blood lymphocyte population is readily measurable. Thus, by measuring a decrease in the level of at least one type of peripheral blood lymphocyte following a depleting event (e.g., the administration of a low ¹³¹I-BC8 dose), one can easily determine that lymphodepletion has occurred in a subject.

"Solid cancers" include, without limitation, bone cancer, pancreatic cancer, skin cancer, cancer of the head or neck, cutaneous or intraocular malignant melanoma, uterine cancer, ovarian cancer, prostate cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, pediatric tumors, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally-induced cancers including those induced by asbestos.

As used herein, the term "subject" includes, without limitation, a mammal such as a human, a non-human primate, a dog, a cat, a horse, a sheep, a goat, a cow, a rabbit, a pig, a rat and a mouse. Where the subject is human, the subject can be of any age. For example, the subject can be 60 years or older, 65 or older, 70 or older, 75 or older, 80 or older, 85 or older, or 90 or older. Alternatively, the subject can be 50 years or younger, 45 or younger, 40 or younger, 35 or younger, 30 or younger, 25 or younger, or 20 or younger. For a human subject afflicted with cancer, the subject can be newly diagnosed, or relapsed and/or refractory, or in remission. "Patient" and "subject" are used interchangeably herein.

As used herein, a "radioisotope" can be an alpha-emitting isotope, a beta-emitting isotope, and/or a gamma-emitting isotope. Examples of radioisotopes include the following: ⁹⁰Y, ⁸⁹Sr, ¹⁵³Sm, ³²P, ²²⁵Ac, ²¹³Bi, ²¹³Po, ²¹¹At, ²¹²Bi, ²¹³Bi, ²²³Ra, ²²⁷Th, ¹⁴⁹Tb, ¹³¹I, ¹³⁷Cs, ²¹²Pb and ¹⁰³Pd. Thus, the radiolabeled antibodies disclosed herein include, without limitation, ⁹⁰Y-antiCD38, ⁸⁹Sr-antiCD38, ¹⁵³Sm-antiCD38, ³²P-antiCD38, ²²⁵Ac-antiCD38, ²¹³Bi-antiCD38, ²¹³Po-antiCD38 , ²¹¹At-antiCD38, ²¹²Bi-antiCD38, ²¹³Bi-antiCD38, ²²³Ra-antiCD38, ²²⁷Th-antiCD38 , ¹⁴⁹Tb-antiCD38, ¹³¹1-antiCD38, ¹³⁷Cs-antiCD38, ²¹²Pb-antiCD38, and ¹⁰³Pd-antiCD38.

As used herein, "treating" a subject afflicted with a cancer shall include, without limitation, (i) slowing, stopping or reversing the cancer's progression, (ii) slowing, stopping or reversing the progression of the cancer's symptoms, (iii) reducing the likelihood of the cancer's recurrence, and/or (iv) reducing the likelihood that the cancer's symptoms will recur. According to certain preferred aspects, treating a subject afflicted with a cancer means (i) reversing the cancer's progression, ideally to the point of eliminating the cancer, and/or (ii) reversing the progression of the cancer's symptoms, ideally to the point of eliminating the symptoms, and/or (iii) reducing or eliminating the likelihood of relapse (i.e., consolidation, which ideally results in the destruction of any remaining cancer cells).

"Therapeutically effective amount" refers to an amount effective, at dosages and for periods of time necessary, to achieve a desired therapeutic result. A therapeutically effective amount may vary according to factors such as the disease state, age, sex, and weight of the individual, and the ability of a therapeutic or a combination of therapeutics to elicit a desired response in the individual. Exemplary indicators of an effective therapeutic or combination of therapeutics include, for example, improved well-being of the patient, reduction in a tumor burden, arrested or slowed growth of a tumor, and/or absence of metastasis of cancer cells to other locations in the body.

"Inhibits growth" refers to a measurable decrease or delay in the growth of a malignant cell or tissue (e.g., tumor) in vitro or in vivo when contacted with a therapeutic or a combination of therapeutics or drugs, when compared to the decrease or delay in the growth of the same cells or tissue in the absence of the therapeutic or the combination of therapeutic drugs. Inhibition of growth of a malignant cell or tissue in vitro or in vivo may be at least about 10%, 20%, 30%, 40%, 50%, 60%, 70%, 80%, 90%, 95%, 96%, 97%, 98%, 99%, or 100%.

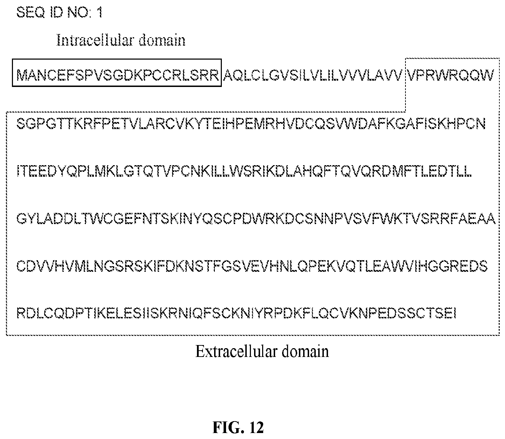

"CD38" refers to the human CD38 protein (synonyms: ADP-ribosyl cyclase 1, cADPr hydrolase 1, Cyclic ADP-ribose hydrolase 1). Human CD38 has the amino acid sequence shown in SEQ ID NO: 1 (**FIG. 12**).

Unless otherwise defined, all technical and scientific terms used herein have the same meaning as commonly understood by one of ordinary skill in the art to which the present invention belongs. Although methods and materials similar or equivalent to those described herein can be used in the practice or testing described herein, suitable methods and materials are described below.

### DETAILED DESCRIPTION

The present invention is related to a composition comprising a radiolabeled monoclonal antibody against CD38 for use as defined in the appended claims. Also disclosed herein are methods for treating a mammal having a hematological malignancy, inhibiting growth and/or proliferation of a cell expressing CD38, and treating a disease or disorder involving cells expressing CD38, by administration of the composition.

Human CD38 has an amino acid sequence shown in GenBank accession number NP_001766 and in SEQ ID NO: 1 (**FIG. 12**). It is well known that CD38 is a single pass type II membrane protein with amino acid residues 1-21 representing the cytosolic domain, amino acid residues 22-42 representing the transmembrane domain, and residues 43-300 representing the extracellular domain of CD38.

Antibodies against CD38, such as daratumumab, MOR202, or SAR650984, have been, and are currently being evaluated in the clinic for their efficacy to treat hematological malignancies and plasma cell disorders, including multiple myeloma. Each antibody has been found to bind to a different portion of the extracellular region of CD38 (Table I), and each demonstrates different clinical responses (*e.g.*, anti-tumor effects). Daratumumab, available from Johnson & Johnson (Janssen Biotech)/Genmab as Darzalex^{®} is described in the publication to de Weers et. al., "Daratumumab, a Novel Therapeutic Human CD38 Monoclonal Antibody, Induces Killing of Multiple Myeloma and Other Hematological Tumors," J Immunology, 2010, 186(3): 1840-1848; MOR202, available from Celgene Corp./Morphosys, is described in U.S. Patent No. 8,877,899; and SAR650984, available from Sanofi/Immunogen as Isatuximab, is described in the publication to Park et al., "SAR650984: A Potent Anti-CD38 Therapeutic Antibody with Three Mechanisms of Action (Apoptosis, ADCC, CDC) for Hematological Malignancies," BLOOD, vol. 112, No. 11, Nov. 2008, p. 951, and Deckert et el., "SAR650984, A Novel Humanized CD38-Targeting Antibody, Demonstrates Potent Antitumor Activity in Models of Multiple Myeloma and Other CD38+ Hematologic Malignancies," Clin Cancer Res 2014; 20:4574-83, and U.S. Patent No. 8,153,765. **Table 1** below lists a number of antibodies, or fragments thereof, that bind CD38, and their epitope (binding site) on the CD38 molecule (*see* **FIG. 12**), some or all of which may be useful according to various aspects disclosed herein.

**TABLE 1**

| Name | Type | **CD38 Epitope** (amino acids) | |
|---|---|---|---|
| | | | Reference |
| SUN4B7 | IgG1 | 254-275 | 1 |
| OKT10 | IgG1 | 280-298 | 1 |
| HB7 | IgG1 | 254-275 | 1, 2 |
| IB4 | IgG2a | 220-241 and 273-285 | 1 |
| IB6 | IgG2b | Not determined | 1 |
| AT1 | IgG1 | 254-275 | 1 |
| SAR650984 or 38SB19 | IgG1 (human) | 107-120, 125, 146, 155, 189, 193, 194 and 226 | 3 |
| Daratumumab | IgG1 (human) | 233-246 and 267-280 | 4 |
| MOR202 | IgG1 (human) | Not determined | 5 |

| | | | |
|---|---|---|---|
| 1) Tissue Antigens 2000, (56):539-547 and BMC Immunology 2004, (5):21 2) J. Biol. Chem. 2011, (286):22170-22177 3) U.S. Pat. No. 8,153,765 and Clin Cancer Res 2014, 20(17):4574-83 4) J Immunol 2011, (186):1840-1848 5) U.S. Pat. No. 8,877,899 | | | |

Proposed methods by which these antibodies eliminate CD38-positive cells include antibody-dependent cellular cytotoxicity (ADCC), complement-dependent cytotoxicity (CDC), and apoptosis.

"Antibody-dependent cellular cytotoxicity", "antibody-dependent cell-mediated cytotoxicity" or "ADCC" is a mechanism for inducing cell death that depends upon the interaction of antibody-coated target cells with effector cells possessing lytic activity, such as natural killer (NK) cells, monocytes, macrophages and neutrophils via Fc gamma receptors (FcγR) expressed on effector cells. For example, NK cells express FcγRIIIa, whereas monocytes express FcγRI, FcγRII and FcvRIIIa. Death of the antibody-coated target cell, such as CD38-expressing cells, occurs as a result of effector cell activity through the secretion of membrane pore-forming proteins and proteases.

"Complement-dependent cytotoxicity", or "CDC", refers to a mechanism for inducing cell death in which an Fc effector domain of a target-bound antibody binds and activates complement component C1q, which in turn activates the complement cascade leading to target cell death. Activation of complement may also result in deposition of complement components on the target cell surface that facilitate ADCC by binding complement receptors (e.g., CR3) on leukocytes.

"Apoptosis" refers to a mechanism of programmed cell death wherein antibody binding to the target cell disrupts integral cell signaling pathways and results in cell self-destruction.

To assess ADCC activity of an antibody that specifically binds CD38, the antibody may be added to CD38-expressing cells in combination with immune effector cells, which may be activated by the antigen-antibody complexes resulting in cytolysis of the CD38-expressing cells. Cytolysis is generally detected by the release of a label (e.g. radioactive substrates, fluorescent dyes or natural intracellular proteins) from the lysed cells. Exemplary effector cells for such assays include peripheral blood mononuclear cells (PBMC) and NK cells.

In an exemplary assay for ADCC, CD38-expressing cells may be labeled with ⁵¹Cr and washed extensively. Anti-CD38 antibodies may be added to the CD38-expressing cells at various concentrations, and the assay started by adding effector cells (NK cells from peripheral blood mononuclear cells, for example). After incubation for various time intervals at 37°C, assays are stopped by centrifugation and ⁵¹Cr release from lysed cells is measured in a scintillation counter. Percentage of cellular cytotoxicity may be calculated as % maximal lysis which may be induced by adding 3% perchloric acid to the CD38-expressing cells.

In an exemplary assay for cytotoxicity, tetrazolium salt is added to CD38-expressing cells treated with various amounts of anti-CD38 antibodies. In living mitochondria, the XTT is reduced to an orange product by mitochondrial dehydrogenase and transferred to the cell surface. The orange product can be optically quantified and reflects the number of living cells. Alternatively, esterases from living cells are known to hydrolyze the colorless calcenin into as fluorescent molecule. The fluorescence can be measured and quantified, and reflects the number of living cells in the sample. The total amount of dead cells may be measured using propidium iodide, which is excluded from live cells by intact membranes. The fluorescence due to the propidium iodide in dead cells may be quantified by flow-cytometry.

In order to assess CDC, complement protein, such as C1q as detailed in Example 2 herein, may need to be included in an assay for cytotoxicity. Measurement of apoptosis induction does not require addition of NK cells or complement protein in an assay for cytotoxicity.

Treatment of various hematological malignancies with unlabeled (*i.e.,* "naked") monoclonal antibodies against CD38 has found success in recent clinical trials. One such antibody, daratumumab, is currently marketed as a treatment for multiple myeloma under the name Darzalex^{®} by Janssen Biotech, Inc. The treatment regime includes an initial 8 week dosing schedule of 1 dose weekly by infusion of 16mg/kg patient weight. Pretreatment with antipyretics and antihistamines are meant to ameliorate some of the more common and serious side effects of infusion. For example, infusion reactions that occur in greater than half of all patients include bronchospasms, hypoxia, dyspnea, hypertension, laryngeal edema, and pulmonary edema, and can occur up to 48 hours after treatment. Side effects that occur in greater than 20 percent of treated patients include at least fatigue, nausea, diarrhea, constipation, muscle spasms, back pains, chills, insomnia, cough, and dyspnea. Thus, while treatment with a naked antibody against CD38 has found some success for the treatment of a hematological malignancy, the side effects of the treatment are fairly serious and may be related to the high protein dose of the monoclonal antibody.

B-cells such as plasma cells and lymphocytes are inherently sensitive to radiotherapy, and are therefore attractive targets for radioimmunotherapies targeted toward hematological malignancies. In addition to the many mechanisms by which anti-CD38 antibodies may kill B-cells, as indicated above, emission of ionizing radiation from a radionuclide labelled antibody against CD38 may kill cells in close proximity to the antibody bound CD38 expressing cells. The radionuclide emits radioactive particles which can damage cellular DNA to the point where the cellular repair mechanisms are unable to allow the cell to continue living. Thus, if the CD38 expressing cells are involved in a tumor, the radionuclide may beneficially kill the tumor cells.

As such, an approach to improve the ability of CD38-targeted immunotherapies to treat hematological malignancies includes labelling the antibody with a radionuclide. On treatment of a patient with the radionuclide labelled monoclonal antibody against CD38, the radionuclide may become localized to cells expressing CD38, such as at a tumor site, and kill those tumor cells.

Radionuclides that can be used to induce such damage to cells, such as cancer cells, are generally high energy emitters. The high energy radionuclide preferably acts over a short range so that the cytotoxic effects are localized to the targeted cells. In this way, radiotherapy is delivered in a more localized fashion to decrease damage to non-targeted or non-cancerous cells.

Thus, the present invention relates to compositions for use as defined in the appended claims, comprising a monoclonal antibody against CD38, wherein the antibody is labeled with a radionuclide. According to the invention, the monoclonal antibody against CD38 is daratumumab.

According to the invention, the radionuclide is the alpha-emitting radionuclide Actinium-225 (²²⁵Ac).

The radionuclide actinium (²²⁵Ac) is a pure alpha-emitter with a half-life of 10 days. It decays via a cascade of six relatively short-lived radionuclide daughters to stable ²⁰⁹Bi. The predominant decay path of ²²⁵Ac yields net four alpha particles with a large cumulative energy of 28 MeV and two beta disintegrations having maximum energy of 1.6 and 0.6 MeV. Its relatively long half-life of 10 days and the multiple alpha particles generated in the rapid decay chain render ²²⁵Ac a highly cytotoxic radionuclide.

The ²²⁵Ac payload conjugated to a monoclonal antibody, antibody fragment, or small peptide ligand can deliver high energy alpha particles directly to the tumor site, generating lethal double strand DNA breaks without the necessitating significant payload accumulation within the tumor cell. Due to its short path length, the range of its high energy alpha particle emission is only a few cell diameters thick, thereby limiting damage to nearby normal tissues. Furthermore, ²²⁵Ac-antibody conjugates offer a crucial advantage over antibody-drug conjugates as they are found to be effective even in patients with low target antigen expressing tumors. This is because of the large cytocidal effects of the ²²⁵Ac, which is in striking contrast to antibody-drug conjugates where hundreds of antibody molecules are needed to bind to their respective antigens to exert effect on a cancer cell.

Other advantages of radioactive payload over drugs or toxins are: 1) The antibody used for radiation delivery does not need to be internalized to kill the cell; 2) Not every cancer cell in the tumor needs to be targeted by the antibody because of the "cross-fire" effect of the radiation; and 3) In contrast to antibody-drug conjugates, the radioisotope linked to the antibody is unlikely to elicit significant immune responses that would limit subsequent use. Moreover, studies reported herein demonstrate the stability of the ²²⁵Ac labelled antibodies.

The monoclonal antibody may be labelled with the alpha-emitting radionuclide by any means known in the art. According to the invention, the radionuclide is attached or chelated by a chelating agent which is conjugated to the monoclonal antibody, as defined in the appended claims.

The radionuclide labelled monoclonal antibody against CD38 ("radiolabeled anti-CD38"), as described herein, may be prepared by first forming a chelator conjugated anti-CD38 ("conjugated anti-CD38"), and then chelating a radionuclide with the conjugated anti-CD38 to form the radiolabeled anti-CD38. A radionuclide may be chelated by the conjugated anti-CD38 at any time following conjugation.

When forming the radiolabeled anti-CD38 using the methods described herein, the degree of chelation and conjugation is advantageously high. As used herein, the terms "degree of chelation" and "degree of conjugation" may be taken to mean the percentage of the radionuclide bound by the chelant, and the percentage of the chelant that is successfully conjugated with the monoclonal antibody, respectively, divided by the total chelant used in the reactions.

The degree of conjugation when forming the conjugated anti-CD38 of the present reaction is generally greater than 50%, greater than 70%, greater than 90%, greater than 95%, greater than about 96%, greater than about 97%, greater than about 98%, or greater than about 99%.

The degree of chelation of the radionuclide when forming the radiolabeled anti-CD38 of the present reaction is generally greater than 50%, greater than 70%, greater than 90%, greater than 95%, greater than about 96%, greater than about 97%, greater than about 98%, or greater than about 99%.

According to the methods of forming the radiolabeled anti-CD38 described herein, the monoclonal antibody against CD38 may be dissolved in a buffered solution comprising a chelant. The pH may be selected to optimize conditions for conjugation of the chelant with the antibody in a conjugation reaction mixture. The conjugation reaction mixture may comprise a bicarbonate buffer or a phosphate buffer. The conjugation reaction mixture may have a pH of about 8.0 to about 9.2. For example, the conjugation reaction mixture may have a pH of about 8.0, about 8.1, about 8.3, about 8.4, about 8.5, about 8.6, about 8.7, about 8.8, about 8.9, about 9.0, about 9.1, or about 9.2. The temperature of the conjugation reaction mixture may be adjusted to promote conjugation of the chelant with the targeting moiety. For example, the conjugation reaction mixture can be incubated at a temperature of about room temperature, or about 37°C. The conjugation reaction mixture may be incubated for any amount of time sufficient to provide conjugation such as, for example, about 1.5 hours.

The conjugated anti-CD38 may be dissolved in a buffered solution comprising a radionuclide. The pH may be selected to optimize conditions for chelation of the radionuclide with the conjugated anti-CD38 in a chelation reaction mixture. The chelation reaction mixture may comprise gentisic acid. The chelation reaction mixture may have a pH of about 5.5 to about 7.0. For example, the chelation reaction mixture may have a pH of about 5.5, about 5.6, about 5.7, about 5.8, about 5.9, about 6.0, about 6.1, about 6.2, about 6.3, about 6.4, about 6.5, about 6.6, about 6.7, about 6.8, about 6.9 or about 7.0.

The temperature of the chelation reaction mixture may be adjusted to promote chelation of the radionuclide with the conjugated anti-CD38. For example, the chelation reaction mixture may be incubated at a temperature of about 37°C. The chelation reaction mixture may be incubated for about 1.5 hours. After a period of time, the solution may be quenched by the addition of a quenching chelate (e.g. diethylenetriaminepentaacetic acid (DTPA)) and the reaction mixture may be purified. The chelation reaction mixture may be further incubated after addition of the quenching chelate, such as for about 30 minutes at about 37°C after addition of the quenching chelate.

The chelators disclosed herein are compounds which have the dual functionality of sequestering metal ions plus the ability to covalently bind a biological carrier such as an antibody. Numerous chelators are known in the art. Exemplary chelators include, but are not limited to chelators such as S-2-(4-Isothiocyanatobenzyl)-1,4,7,10 tetraazacyclododecanetetraacetic acid (p-SCN-Bn-DOTA), diethylene triamine pentaacetic acid (DTPA); ethylene diamine tetraacetic acid (EDTA); 1,4,7,10-tetraazacyclododecane-N,N',N",N‴-tetraacetic acid (DOTA); p-isothiocyanatobenzyl-1,4,7,10-tetraazacyclododecane-1,4,7,10-te-traacetic acid (p-SCN-Bz-DOTA); 1,4,7,10-tetraazacyclododecane-N,N',N"-triacetic acid (DO3A); 1,4,7,10-tetraazacyclododecane-1,4,7,10-tetrakis(2-propionic acid) (DOTMA); 3,6,9-triaza-12-oxa-3,6,9-tricarboxymethylene-10-carboxy-13-phenyl-tridecanoic acid ("B-19036"); 1,4,7-triazacyclononane-N,N',N"-triacetic acid (NOTA); 1,4,8,11-tetraazacyclotetradecane-N,N',N",N"'-tetraacetic acid (TETA); triethylene tetraamine hexaacetic acid (TTHA); trans-1,2-diaminohexane tetraacetic acid (CYDTA); 1,4,7,10-tetraazacyclododecane-1-(2-hydroxypropyl)-4,7,10-triacetic acid (HP-DO3A); trans-cyclohexane-diamine tetraacetic acid (CDTA); trans(1,2)-cyclohexane dietylene triamine pentaacetic acid (CDTPA); 1-oxa-4,7,10-triazacyclododecane-N,N',N"-triacetic acid (OTTA); 1,4,7,10-tetraazacyclododecane-1,4,7,10-tetrakis{3-(4-carboxyl)-butanoic acid}; 1,4,7,10-tetraazacyclododecane-1,4,7,10-tetrakis(acetic acid-methyl amide); 1,4,7,10-tetraazacyclododecane-1,4,7,10-tetrakis(methylene phosphonic acid); and derivatives thereof.

One or more steps may be used to separate the conjugated CD38 from other constituents of the conjugation reaction mixture or the radiolabeled anti-CD38 from other constituents of the chelation reaction mixture. For example, the reaction mixture can be transferred to a filtering device (*e.g.,* a Millipore centrifugal device) having a particular molecular weight cut off such that filtration of the reaction mixture through the filtration device can separate the conjugated anti-CD38 or the radiolabeled anti-CD38 from other constituents of the respective reaction mixture. Filtration can be used to obtain a conjugated anti-CD38 or a the radiolabeled anti-CD38 having at least about 60%, at least about 65%, at least about 70%, at least about 75%, at least about 80%, at least about 85%, at least about 90%, %, at least about 95%, at least about 97%, at least about 98%, at least about 99%, or at least about 99.5% purity.

According to certain aspects of the invention, the yield of the conjugated anti-CD38 or the radiolabeled anti-CD38 from the separation (*e.g.,* purification) is at least about 70%, at least about 75%, at least about 80%, at least about 85%, or at least about 90%, at least 95%, at least 96%, at least 97%, at least 98%, or at least 99% of the final product.

According to one aspect of the present invention, the monoclonal antibody may be first conjugated with a p-SCN-Bn-DOTA or DOTA chelating agent to form the conjugated anti-CD38, followed by chelation of ²²⁵Ac by the p-SCN-Bn-DOTA or DOTA on the conjugated anti-CD38 to form the radiolabeled anti-CD38. Thus, according to aspects of the present invention, only a single step involving ²²⁵Ac is needed to label the anti-CD38.

According to certain aspects of the present invention, the radionuclide (²²⁵Ac) may be chelated by the chelating agent prior to conjugation with the monoclonal antibody against CD38.

According to certain aspects of the present invention, the radiolabeled anti-CD38 is relatively stable. For example, greater than 75% of the actinium labelled monoclonal antibody may remain intact after storage for 24 hours at 4°C.

According to certain aspects of the present invention, the radiolabeled anti-CD38 exhibits essentially the same immunoreactivity to CD38 as a control monoclonal antibody, wherein the control monoclonal antibody comprises an unlabeled monoclonal antibody against the same epitope of CD38 as the actinium labelled monoclonal antibody.

The labelling efficiency, stability of the radiolabeled anti-CD38, and immunoreactivity of the labelled CD38 combine to provide an effective therapeutic agent. As such, the radiolabeled anti-CD38 produced using the methods described herein may be used as a therapeutic agent. For example, the radiolabeled anti-CD38 may be used as a therapeutic agent that delivers doses of radiation specifically to CD38 expressing cells and tissues. Thus, according to certain aspects of the present invention, the radiolabeled anti-CD38 may be formulated in solution with a pharmaceutically acceptable salt or carrier to form a pharmaceutical composition.

The pharmaceutical compositions of the present invention may also be administered in combination therapy, i.e., combined with other therapeutic agents relevant for the disease or condition to be treated. Such administration may be simultaneous, separate or sequential. For simultaneous administration, the agents may be administered as one compositions or as separate compositions, as appropriate.

According to certain aspects of the present invention, the pharmaceutical composition may include one or more therapeutic agents. Exemplary therapeutic agents include a chemotherapeutic agent, an anti-inflammatory agent, an immunosuppressive, an immunomodulatory agent, or a combination thereof.

Therapeutic agents may be administered according to any standard dose regime known in the field. When therapeutic agents are included in the composition of the present invention, they may be included at concentrations in the range of 1 to 500 mg/m², the amounts being calculated as a function of patient surface area (m²). For example, exemplary doses of paclitaxel may include 15 mg/m² to 275 mg/m², exemplary doses of docetaxel may include 60 mg/m² to 100 mg/m², exemplary doses of epithilone may include 10 mg/m² to 20 mg/m², and an exemplary dose of calicheamicin may include 1 mg/m² to 10 mg/m². While exemplary doses are listed herein, such are only provided for reference and are not intended to limit the dose ranges of the drug agents of the presently disclosed invention.

Thus, according to one aspect, the pharmaceutical composition may include at least one chemotherapeutic agent. Exemplary chemotherapeutic agents include an antimetabolite, such as methotrexate, 6-mercaptopurine, 6-thioguanine, cytarabine, fludarabine, 5-fluorouracil, decarbazine, hydroxyurea, asparaginase, gemcitabine, cladribine and similar agents.

Exemplary chemotherapeutic agents include an alkylating agent, such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin, and similar agents.

Exemplary chemotherapeutic agents include an antibiotic, such as dactinomycin (formerly actinomycin), bleomycin, calicheamicin, daunorubicin (formerly daunomycin), doxorubicin, idarubicin, mithramycin, mitomycin, mitoxantrone, plicamycin, anthramycin (AMC) and similar agents.

Exemplary chemotherapeutic agents include anti-mitotic agent, such as taxanes, for instance docetaxel, and paclitaxel, and vinca alkaloids, for instance vindesine, vincristine, vinblastine, and vinorelbine.

Exemplary chemotherapeutic agents include a topoisomerase inhibitor, such as topotecan.

Exemplary chemotherapeutic agents include a growth factor inhibitor, such as an inhibitor of ErbB1 (EGFR) (such as gefitinib (Iressa^{®}), cetuximab (Erbitux^{®}), erlotinib (Tarceva^{®}), HuMax-EGFr (2F8 disclosed in WO 2002/100348) and similar agents), an inhibitor of ErbB2 (Her2/neu) (such as trastuzumab (Herceptin^{®}) and similar agents) and similar agents. In one embodiment, such a growth factor inhibitor may be a farnesyl transferase Inhibitor, such as SCH-66336 and R115777. In one embodiment, such a growth factor inhibitor may be a vascular endothelial growth factor (VEGF) inhibitor, such as bevacizumab (Avastin^{®}).

Exemplary chemotherapeutic agents include a tyrosine kinase inhibitor, such as imatinib (Glivec, Gleevec ST1571), lapatinib, PTK787/ZK222584 and similar agents.

Exemplary chemotherapeutic agents include a histone deacetylase inhibitor. Examples of such histone deacetylase inhibitors include hydroxamic acid-based hybrid polar compounds, such as SAHA (suberoylanilide hydroxamic acid).

Exemplary chemotherapeutic agents include a P38a MAP kinase inhibitor, such as SCIO-469.

Exemplary chemotherapeutic agents include inhibitors of angiogenesis, neovascularization, and/or other vascularization. Examples of such inhibitors include urokinase inhibitors, matrix metalloprotease inhibitors (such as marimastat, neovastat, BAY 12-9566, AG 3340, BMS-275291 and similar agents), inhibitors of endothelial cell migration and proliferation (such as TNP-470, squalamine, 2-methoxyestradiol, combretastatins, endostatin, angiostatin, penicillamine, SCH66336 (Schering-Plough Corp, Madison, N.J.), R115777 (Janssen Pharmaceutica, Inc, Titusville, N.J.) and similar agents), antagonists of angiogenic growth factors (such as such as ZD6474, SU6668, antibodies against angiogenic agents and/or their receptors (such as VEGF, bFGF, and angiopoietin-1), thalidomide (Thalomid^{®}), thalidomide analogs (such as CC-5013 (lenalidomide, Revlimid^{™}) and CC4047 (Actimid^{™}), Sugen 5416, SU5402, antiangiogenic ribozyme (such as angiozyme), interferon α (such as interferon α2α), suramin and similar agents), VEGF-R kinase inhibitors and other anti-angiogenic tyrosine kinase inhibitors (such as SU011248), inhibitors of endothelial-specific integrin/survival signaling (such as vitaxin and similar agents), copper antagonists/chelators (such as tetrathiomolybdate, captopril and similar agents), carboxyamido-triazole (CAI), ABT-627, CM101, interleukin-12 (IL-12), IM862, PNU145156E as well as nucleotide molecules inhibiting angiogenesis (such as antisense-VEGF-cDNA, cDNA coding for angiostatin, cDNA coding for p53 and cDNA coding for deficient VEGF receptor-2) and similar agents.

Other examples of such inhibitors of angiogenesis, neovascularization, and/or other vascularization are anti-angiogenic heparin derivatives and related molecules (e.g., heperinase III), temozolomide, NK4, macrophage migration inhibitory factor (MIF), cyclooxygenase-2 inhibitors, inhibitors of hypoxia-inducible factor 1, anti-angiogenic soy isoflavones, oltipraz, fumagillin and analogs thereof, somatostatin analogues, pentosan polysulfate, tecogalan sodium, dalteparin, tumstatin, thrombospondin, NM-3, combrestatin, canstatin, avastatin, antibodies against other relevant targets (such as anti-alpha-v/beta-3 integrin and anti-kininostatin mAbs) and similar agents.

Exemplary chemotherapeutic agents include thalidomide (Thalomid^{®}), thalidomide analogs (such as CC-5013 (lenalidomide, Revlimid^{™}) and/or CC4047 (Actimid^{™}),

Exemplary chemotherapeutic agents may include additional antibody therapeutics, or drugs such as a proteasome inhibitor, such as bortezomib (Velcade^{®}), a corticosteroid, such as prednisone, prednisolone, dexamethasone, etc, a bisphosphonate. Examples of potentially suitable biphosphonates are pamidronate (Aredia^{®}), zoledronic acid (Zometa^{®}), clodronate (Bonefos^{®}), risendronate (Actonel^{®}), ibandronate (Boniva^{®}), etidronate (Didronel^{®}), alendronate (Fosamax^{®}), tiludronate (Skelid^{®}), incadronate (Yamanouchi Pharmaceutical) and minodronate (YM529, Yamanouchi).

Exemplary chemotherapeutic agents include a colony stimulating factor. Examples of suitable colony stimulating factors are granulocyte-colony stimulating factors (G-CSF), such as filgrastim (Neupogen^{®}) and pegfilgrastim (Neulasta^{®}), and granulocyte macrophage-colony stimulating factors (GM-CSF) such as sargramostim (Leukine^{®}).

Exemplary chemotherapeutic agents include an erythropoietic agent. Examples of suitable erythropoietic agents are erythropoietin (EPO), such as epoetin alfa (for instance Procrit^{®}, Epogen^{®}, and Eprex^{®}) and epoetin beta (for instance NeoRecormon^{®}) and erythropoiesis-stimulating proteins (for instance Aranesp^{®}).

Exemplary chemotherapeutic agents include an anti-anergic agents (for instance small molecule compounds, proteins, glycoproteins, or antibodies that break tolerance to tumor and cancer antigens).

Exemplary chemotherapeutic agents include a virus, viral proteins, and the like. Replication-deficient viruses, that generally are capable of one or only a few rounds of replication in vivo, and that are targeted to tumor cells, may for instance be useful components of such compositions and methods. Such viral agents may comprise or be associated with nucleic acids encoding immunostimulants, such as GM-CSF and/or IL-2. Both naturally oncolytic and such recombinant oncolytic viruses (for instance HSV-1 viruses, reoviruses, replication-deficient and replication-sensitive adenovirus, etc.) may be useful components of such methods and compositions.

According to another aspect, the pharmaceutical composition may include an anti-inflammatory agent may be selected from a steroidal drug and a NSAID (nonsteroidal anti-inflammatory drug). Other anti-inflammatory agents may be selected from aspirin and other salicylates, Cox-2 inhibitors (such as rofecoxib and celecoxib), NSAIDs (such as ibuprofen, fenoprofen, naproxen, sulindac, diclofenac, piroxicam, ketoprofen, diflunisal, nabumetone, etodolac, oxaprozin, and indomethacin), anti-IL6R antibodies, anti-IL8 antibodies, anti-IL15 antibodies, anti-IL15R antibodies, anti-CD4 antibodies, anti-CD11a antibodies (e.g., efalizumab), anti-alpha4/beta-1 integrin (VLA4) antibodies (e.g natalizumab), CTLA4-1 g for the treatment of inflammatory diseases, prednisolone, prednisone, disease modifying antirheumatic drugs (DMARDs) such as methotrexate, hydroxychloroquine, sulfasalazine, pyrimidine synthesis inhibitors (such as leflunomide), IL-1 receptor blocking agents (such as anakinra), TNF-α blocking agents (such as etanercept, infliximab, and adalimumab) and similar agents.

According to another aspect, the pharmaceutical composition may include at least one immunosuppressive and/or immunomodulatory agent to a subject in need thereof. Examples of an immunosuppressive and/or immunomodulatory agent include cyclosporine, azathioprine, mycophenolic acid, mycophenolate mofetil, corticosteroids such as prednisone, methotrexate, gold salts, sulfasalazine, antimalarials, brequinar, leflunomide, mizoribine, 15-deoxyspergualine, 6-mercaptopurine, cyclophosphamide, rapamycin, tacrolimus (FK-506), OKT3, anti-thymocyte globulin, thymopentin, thymosin-α and similar agents.

Additional immunosuppressive and/or immunomodulatory agents may be selected from immunosuppressive antibodies, such as antibodies binding to p75 of the IL-2 receptor, or antibodies binding to for instance MHC, CD2, CD3, CD4, CD7, CD28, B7, CD40, CD45, IFNγ, TNF-α, IL-4, IL-5, IL-6R, IL-6; IGF, IGFR1, IL-7, IL-8, IL-10, CD11a, or CD58, or antibodies binding to their ligands. Further additional immunosuppressive and/or immunomodulatory agents may be selected from soluble IL-15R, IL-10, B7 molecules (B7-1, B7-2, variants thereof, and fragments thereof, ICOS, and OX40, an inhibitor of a negative T cell regulator (such as an antibody against CTLA4) and similar agents.

According to certain aspects of the present invention, the one or more therapeutic agents comprises an antimyeloma agent. Exemplary antimyeloma agents include dexamethasone, melphalan, doxorubicin, bortezomib, lenalidomide, prednisone, carmustine, etoposide, cisplatin, vincristine, cyclophosphamide, and thalidomide, several of which are indicated above as chemotherapeutic agents, anti-inflammatory agents, or immunosuppressive agents.

According to the present invention, the pharmaceutical composition further comprises a non-radiolabeled monoclonal antibody against an epitope of CD38, which is daratumumab. The amount of non-radiolabeled anti-CD38 included in the pharmaceutical compositions may vary depending on the exact nature of the disease to be treated, age and weight of the patient, identity of the monoclonal antibody against CD38, and the radionuclide selected for labelling of the monoclonal antibody.

According to certain aspects of the present invention, the pharmaceutical composition may comprise the non-radiolabeled monoclonal antibody in an amount that is at least equal to the amount of the radiolabeled anti-CD38 in the pharmaceutical composition, or in an amount that is at least 2-fold greater, such as at least 4-fold greater, or at least 10-fold greater, than the amount of the radiolabeled anti-CD38 in the pharmaceutical composition.

According to certain aspects of the present invention, the radiolabeled anti-CD38 may be included in the compositions or pharmaceutical composition detailed herein at 0.1ug/ml or less, such as 0.06ug/ml or less, or 0.04ug/ml or less, or even 0.02 ug/ml or less.

When included in such doses, the composition or pharmaceutical compositions detailed herein may cause greater than 50% cell death of lymphoblast or myeloma cells within 72 hours of administration, or 70% cell death of lymphoblast or myeloma cells within 96 hours of administration, or 90% cell death of lymphoblast or myeloma cells within 96 hours of administration, or even 95% cell death of lymphoblast or myeloma cells within 96 hours of administration.

According to certain aspects of the present invention, the actinium labelled monoclonal antibody may be at least 5-fold more effective at causing cell death of lymphoblast or myeloma cells than a control monoclonal antibody, wherein the control monoclonal antibody comprises an unlabeled monoclonal antibody against the same epitope of CD38 as the actinium labelled monoclonal antibody. For example, the actinium labelled monoclonal antibody may be at least 10-fold more effective, at least 20-fold more effective, at least 50-fold more effective, or at least 100-fold more effective at causing cell death of lymphoblast or myeloma cells than the control monoclonal antibody.

Maximal lysis via CDC and CDCC using Darzalex^{®}, an unlabeled anti-CD38 therapeutic currently on the market, has been reported in the literature to occur at 1ug/ml and 0.10ug/ml, respectively. As indicated above, the actinium labelled monoclonal antibody may be at least 5-fold more effective at causing cell death. Without wishing to be bound by theory, such an increase in the cytotoxicity of the radiolabeled anti-CD38 may include cell death via additional mechanisms, such as, for example, apoptosis.

The pharmaceutical composition of the present invention may be used to treat a hematological malignancy, or to inhibit growth and/or proliferation of a cell expressing CD38, or to treat a disease or disorder involving cells expressing CD38. Disclosed herein are methods for treating a subject having a hematological malignancy, for inhibiting growth and/or proliferation of a cell expressing CD38, and for treating a disease or disorder involving cells expressing CD38, wherein the methods comprise administering to a subject the pharmaceutical composition detailed hereinabove.

According to certain aspects of the present disclosure, the hematological malignancy is multiple myeloma. According to certain aspects of the invention the cell expressing CD38 is a multiple myeloma cell. According to certain aspects of the disclosure, the disease or disorder may be multiple myeloma.

According to the invention the cells expressing CD38 are CD38-expressing cancer cells or CD38-expressing T-cells, B-cells, NK cells, or plasma cells. According to the invention the cells expressing CD38 also comprise solid tumor cells or hematological malignancy cells. Exemplary hematological malignancy cells comprise multiple myeloma cells, acute lymphocytic leukemia cells, acute myeloid leukemia cells, chronic lymphocytic leukemia cells, chronic myeloid leukemia cells, Hodgkin's lymphoma cells, non-Hodgkin's lymphoma cells, T-LGL leukemia cells, NK cell leukemia cells, or hairy cell leukemia cells.

As indicated above, the pharmaceutical composition may be administered either alone or in combination with one or more additional therapeutic agents. The pharmaceutical composition may comprise the one or more additional therapeutic agents. The pharmaceutical composition may be administered in a dosage regime comprising at least one dose. Any reference to a method of treatment practised on the human or animal body is to be interpreted as substances and compositions for use in such treatment.

According to certain aspects of the present invention, the at least one dose of the dosage regimen comprises an amount of the monoclonal antibody against CD38 that is 5-fold lower than a dose in a dosage regime comprising only a control monoclonal antibody. According to certain aspects of the present invention, the at least one dose of the dosage regimen comprises an amount of the monoclonal antibody against CD38 that is 10-fold lower than the dose in the dosage regime comprising only the control monoclonal antibody. According to certain aspects of the present invention, the at least one dose of the dosage regimen comprises an amount of the monoclonal antibody against CD38 that is 20-fold lower than the dose in the dosage regime comprising only the control monoclonal antibody. According to certain aspects of the present invention, the at least one dose of the dosage regimen comprises an amount of the monoclonal antibody against CD38 that is 50-fold lower than the dose in the dosage regime comprising only the control monoclonal antibody. According to certain aspects of the present invention, the at least one dose of the dosage regimen comprises an amount of the monoclonal antibody against CD38 that is 100-fold lower than the dose in the dosage regime comprising only the control monoclonal antibody. The control monoclonal antibody generally comprises an unlabeled monoclonal antibody against the same epitope of CD38 as the actinium labelled monoclonal antibody.

According to certain aspects of the present invention, the dosage regime comprises at least one less dose than a control dosage regime, wherein the control dosage regime includes administration, either alone or in combination with one or more additional therapeutic agents, of an unlabeled monoclonal antibody against the same epitope of CD38 as the actinium labelled monoclonal antibody. According to certain aspects of the present invention, the dosage regime comprises at least two fewer doses than a control dosage regime, wherein the control dosage regime includes administration, either alone or in combination with one or more additional therapeutic agents, of an unlabeled monoclonal antibody against the same epitope of CD38 as the actinium labelled monoclonal antibody.

According to certain aspects of the present invention, the dosage regime may comprise at 10% fewer doses than the control dosage regime, such as 20% fewer doses, or 30% fewer doses, or 40% fewer doses, or 50% fewer doses, or 60% fewer doses, or 70% fewer doses, or 80% fewer doses, or even 90% fewer doses, than the control dosage regime.

According to certain aspects of the present invention, the control monoclonal antibody may comprise daratumumab administered at a dose of about 16 mg/kg patient weight. According to certain aspects of the present invention, the control dosage regime may include 8 doses, administered at one dose per week for at least 8 weeks.

According to certain aspects of the present invention, the therapeutically effective dose of the radiolabeled anti-CD38 antibody may be 0.1ug/kg to 1mg/kg, such as 1ug/kg to 1mg/kg, or 10ug/kg to 1mg/kg, or 100ug/kg to 1mg/kg, or 0.1ug/kg to 100ug/kg, or 0.1ug/kg to 50ug/kg, or 0.1ug/kg to 10ug/kg, or 0.1ug/kg to 40ug/kg, or 1ug/kg to 40ug/kg.

These protein doses may include a radioactive dose of 0.1uCi/kg to 5uCi/kg, such as 0.1uCi/kg to 4uCi/kg, or 0.1uCi/kg to 3uCi/kg, or 0.1uCi/kg to 2uCi/kg, or 0.1uCi/kg to 1uCi/kg, or 0.2uCi/kg to 5uCi/kg, or 0.5uCi/kg to 5uCi/kg.

The therapeutically effective dose of the radiolabeled anti-CD38 antibody may be administered in a single dose, or as two equal fractionated doses, wherein a second dose may be administered 1 day to 10 days, such as 3-8 days or 4-7 days or 5-8 days, after the first dose.

Also disclosed herein are methods for treating a cancer in a subject, wherein the method comprises administering to the subject a therapeutically effective amount of the pharmaceutical composition detailed hereinabove. Without wishing to be bound by any particular theory, based on the immunomodulatory effects observed with the anti-CD38 monoclonal antibody described herein, such as daratumumab, MOR202, or SAR650984, may be efficacious in treatment of solid tumors. Thus, also disclosed herein is a method of treating a patient having a solid tumor, comprising administering to the patient in need thereof a therapeutically effective amount of an antibody that specifically binds CD38 for a time sufficient to treat the solid tumor.

According to certain aspects, the present invention provides articles of manufacture as defined in the appended claims. For example, an article of manufacture according to aspects of the present invention comprises (a) a radiolabeled monoclonal antibody against CD38, and (b) a label instructing a user to administer to a subject an amount of the antibody effective to treat a disease or disorder involving cells expressing CD38. The monoclonal antibody is daratumumab, wherein the article of manufacture comprises daratumumab labeled with actinium (²²⁵Ac). According to further aspects of the article of manufacture, the amount of the antibody effective to treat the disease or disorder involving cells expressing CD38 comprises 10µCi to 600µCi of the ²²⁵Ac-labelled daratumumab, such as 10µCi to 400µCi of the ²²⁵Ac-labelled daratumumab, or 10µCi to 200µCi of the ²²⁵Ac-labelled daratumumab.

According to the present invention, the article of manufacture comprises (a) a pharmaceutical composition as defined in the appended claims, and (b) a label instructing a user to administer to a subject an amount of the antibody effective to treat a disease or disorder involving cells expressing CD38. The pharmaceutical composition comprises a radiolabeled fraction of the monoclonal antibody against CD38, and an unlabeled fraction of the same monoclonal antibody against CD38. The pharmaceutical composition may be provided in a patient specific form, such as at a therapeutically effective dose comprising an amount of radioactivity and a protein concentration that are tailored for a specific patient (e.g., based on patient characteristics such as weight, sex, age, disease type and progression, etc.). According to certain aspects, the therapeutically effective dose may comprise radiolabeled anti-CD38 antibody at 0.1ug/kg to 1mg/kg, such as 1ug/kg to 1mg/kg, or 10ug/kg to 1 mg/kg, or 100ug/kg to 1 mg/kg, or 0.1 ug/kg to 100ug/kg, or 0.1 ug/kg to 50ug/kg, or 0.1ug/kg to 10ug/kg, or 0.1ug/kg to 40ug/kg, or 1ug/kg to 40ug/kg; having a radioactive dose of 0.1uCi/kg to 5uCi/kg, such as 0.1uCi/kg to 4uCi/kg, or 0.1uCi/kg to 3uCi/kg, or 0.1uCi/kg to 2uCi/kg, or 0.1uCi/kg to 1uCi/kg, or 0.2uCi/kg to 5uCi/kg, or 0.5uCi/kg to 5uCi/kg.

According to certain aspects, the present invention provides a pharmaceutical composition useful for treatment of a disease or disorder involving cells expressing CD38, as defined in the appended claims. The composition may comprise 5 to 50 wt.% of the monoclonal antibody against CD38 labeled with actinium (²²⁵Ac); 50 to 95 wt.% of the monoclonal antibody against CD38 unlabeled; and a pharmaceutically acceptable carrier. The radiolabeled monoclonal antibody is ²²⁵Ac-daratumumab, and the unlabeled monoclonal antibody is the same as the labelled antibody, i.e., daratumumab.

### EXAMPLES

### EXAMPLE 1: Radiolabeling of daratumumab

Daratumumab (DARA) manufactured by Janssen Biotech (USA) was purchased from the pharmacy at the Montefiore Medical Center, New York, USA. Isotype control human IgG1 was purchased from Creative Diagnotics (New York, USA). ²²⁵Ac in dry nitrate form was obtained from Oak Ridge National Laboratory, USA. Indium-111 (¹¹¹In) in form of ¹¹¹In chloride was purchased from MDS Nordion (Vancouver, BC, Canada). The CD38-positive lymphoma cell line Daudi was procured from ATCC (Manassas, USA), and CD38-positive multiple myeloma cell lines KMS-28BM and KMS-28PE from XenoTech (Japan). The Daudi cell line was grown in RPMI, 10% FBS and antimicrobial cocktail, and KMS-28BM and KMS-28PE cell lines were grown in RPMI supplemented with 10% FBS. Bifunctional chelating agent p-SCN-Bn-DOTA (referred to as DOTA in these examples) was purchased from Macrocyclics (Texas, USA).

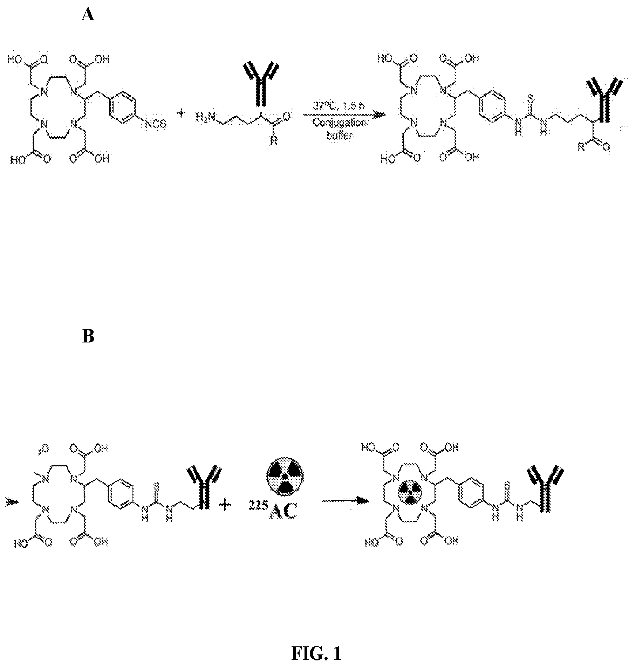

With reference to **FIGS. 1A** and **1B****,** DOTA was conjugated to DARA at 5M excess for 1.5h at 37°C in ammonium acetate buffer (**FIG. 1A**). The DARA-DOTA conjugate was labeled with ²²⁵Ac or ¹¹¹In at a specific activity of 400nCi to 0.3µg to produce ²²⁵A_{c}-DARA (**FIG. 1B**). The ²²⁵Ac-DARA was diluted with an equal amount of unlabeled DARA to adjust for total antibody dose (0.3µg) for the 200uCi ²²⁵Ac-DARA dose. The samples were subsequently purified on disposable spin columns to99±1 radiochemical purity.

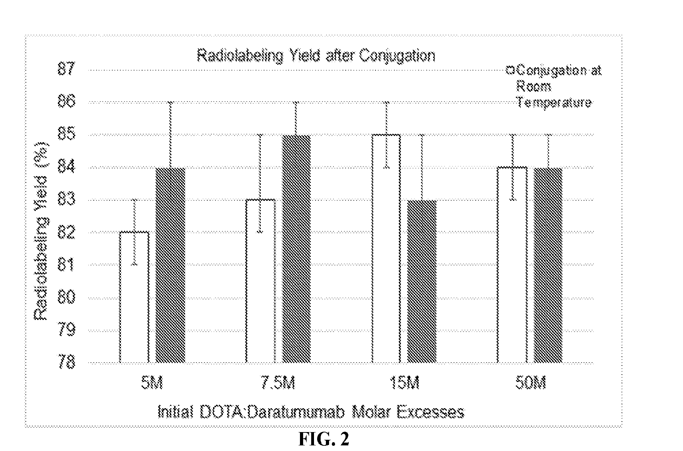

**FIG. 2** provides a bar graph that shows the degree of chelation of ²²⁵Ac by the DOTA-DARA immunoconjugate, wherein the conjugation reaction was performed at 37°C or room temperature, and at various molar excesses of the DOTA chelator to the DARA. The DOTA-DARA immunoconjugate at 0.3 mg/ml in 0.15M ammonium acetate buffer, pH 6.5 was labelled with ²²⁵Ac (chelation of ²²⁵Ac by DOTA) in 0.01M HCL at 1uCi/ug daratumumab for 60 minutes at 37°C or room temperature.

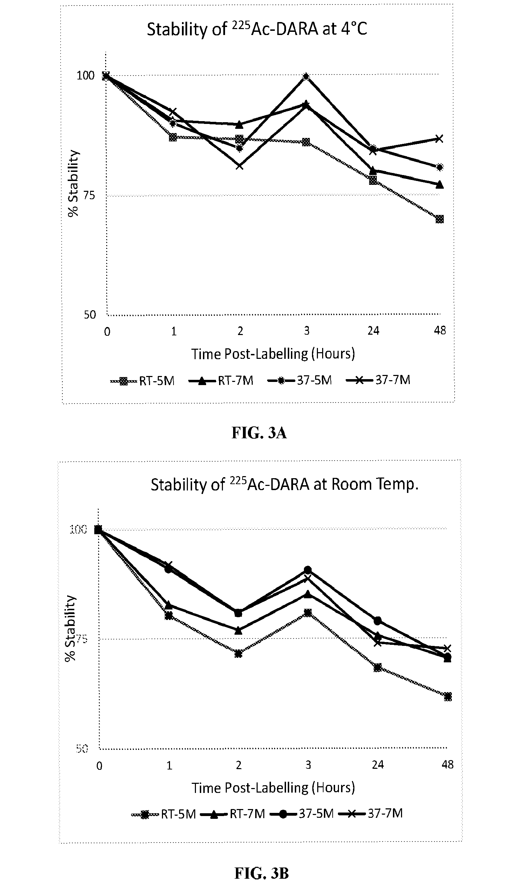

Stability of the ²²⁵Ac-DARA was measured on storage at two different temperatures. **FIG. 3A** shows the stability on storage at 4°C of various ²²⁵Ac-DARA samples, wherein the samples are from conjugation reactions between DOTA and DARA performed at 37°C or room temperature, and at various molar excesses of DOTA to DARA. **FIG. 3B** shows the stability on storage at room temperature of various ²²⁵Ac-DARA samples, wherein the samples are from conjugation reactions between DOTA and DARA performed at 37°C or room temperature, and at various molar excesses of DOTA to DARA. Samples were purified on disposable spin column to 99 ± 1% before the start of the stability experiments. At both temperatures, greater than 75% of the actinium labelled monoclonal antibody remains intact after storage for 24 hours at 4°C, and greater than 60% of the actinium labelled monoclonal antibody remains intact after storage for 24 hours at room temperature.

### EXAMPLE 2: Immunoreactivity of the ²²⁵Ac-DARA

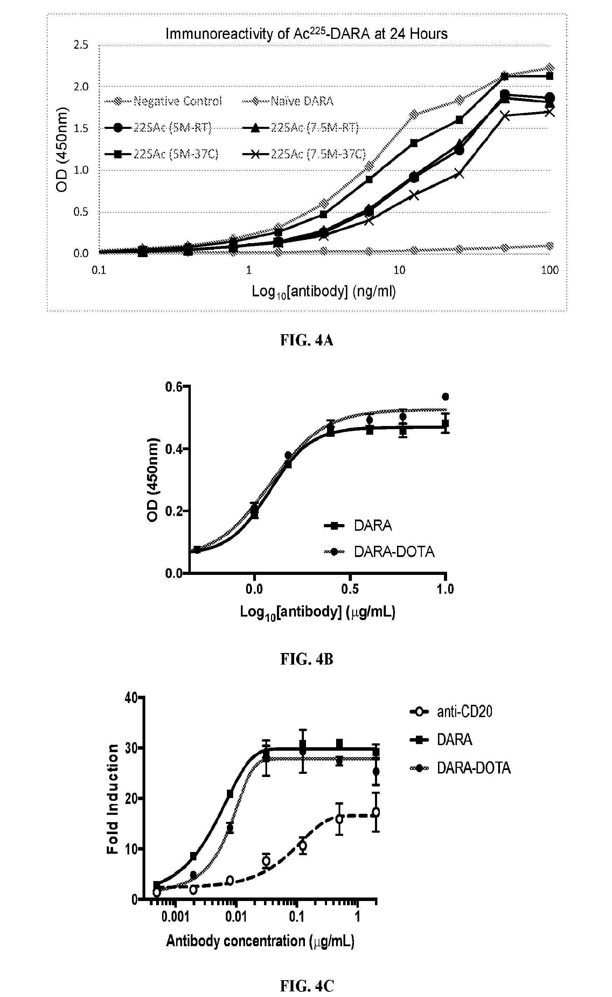

As shown in **FIG. 4A****,** binding of ²²⁵Ac-DARA conjugates to various concentrations of CD38 is essentially unchanged when compared to an unlabeled anti-CD38, even for ²²⁵Ac-DARA conjugates from conjugation reactions between DOTA and DARA performed at 37°C or room temperature, and at various molar excesses of DOTA to DARA.

The binding of the complement component 1q (C1q) to DARA and DOTA conjugated daratumumab was assessed using an ELISA binding assay. As shown in **FIG. 4B****,** conjugation of the DARA to DOTA does not affect complement binding. High binding 96-well plates (Corning) were coated overnight at 4 °C with varying concentrations of antibody in coating buffer (100 mM sodium carbonate, pH 9.6). After each incubation, sample wells were washed three times with PBST (0.05% Tween 20/PBS, pH 7.4). After coating, plates were blocked for one hour at RT with blocking buffer (0.1% BSA/PBST) that was then replaced with 2 µg/mL human C1q in blocking buffer. After one hour, wells were washed three times and 100 µL of 1 µg/mL anti-hC1qHRP in blocking buffer was added. After one hour wells were washed and 100 µL TMB substrate (Pierce, Rockford, IL) was added. After 15 min the reaction was stopped with 100 µL 1M HCl and the absorbance at 450 nm was read with a Spectra MAX 250 plate reader (Molecular Devices, San Jose, CA).

The ability of DARA and DOTA conjugated DARA to mediate ADCC was assessed using the ADCC Reporter Bioassay, Complete Kit (Raji) purchased from Promega (Madison,WI) and used according to manufacturer's protocol. As shown in **FIG. 4C****,** various concentrations of DARA and DARA-DOTA were added to CD38 expressing target cells. Effecter cells that express luciferase when activated through FcγR(III) were added to target cells and luminescence was measured after addition of Bio-Glow^{™}. Fold induction = RLU(inducedbackground)/RLU(no antibody control-background). Anti-CD20 antibody, which is known to activate ADCC, was used as a positive control. As shown, conjugation of DARA to DOTA does not inhibit the ADCC.

### EXAMPLE 3: Cytotoxicity of the ²²⁵Ac-DARA

The ability of the naked DARA, ²²⁵Ac-DARA, and irrelevant IgG-²²⁵Ac to induce cell lysis in multiple myeloma cell lines with a range of CD38 antigen expression levels was assessed at various time points and concentrations.

Daudi cells (2x10⁵ in 1 mL PBS) were placed in BSA-blocked Eppendorf tubes and treated in triplicate with 0, 20, 40, 60 and 100 nCi/sample with ²²⁵Ac-DARA, or the matching activities of the control human IgG, or unlabeled DARA. The total amount of DARA per 1 mL sample was 0.02 µg for 20 nCi samples, 0.04 µg for 40 nCi samples, 0.06 µg for 60 nCi samples and for matching samples of unlabeled DARA. After 48, 72 and 96 hrs incubation, the effect of the radiolabeled antibodies on the cells was evaluated using the tetrazolium dye (2,3)-bis-(2-methoxy-4-nitro-5-sulphenyl)-(2H)-terazolium-5-carboxanilide (XTT) assay. Wells were washed, fresh media added along with 50 µl XTT (Sigma) at 1 mg/ml in PBS, and 4 µl menadione (Sigma) at 1 mM in acetone. Cells were incubated for another 3 hrs and the absorbance at 492 nm was read. All conditions were performed in triplicate. In parallel, the killing was evaluated with Trypan blue staining.

Shown in **FIGS. 5A-5D** are results from the XTT assay for treatment of Daudi cells with ²²⁵Ac-DARA conjugates. It can be seen that lysis of Daudi cells by ²²⁵Ac-DOTA-IgG conjugates approaches 95% to 100%, while the control DARA shows little to no effect at the low concentrations tested in the assay (i.e., same cytotoxicity as the control cell). **FIGS. 5A-5D** show exposure times of 24, 48, 72, and 96 hours, respectively.

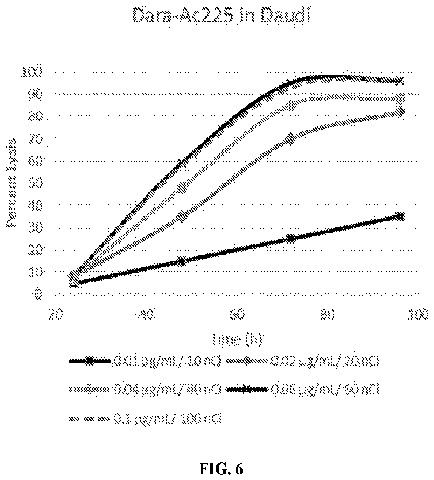

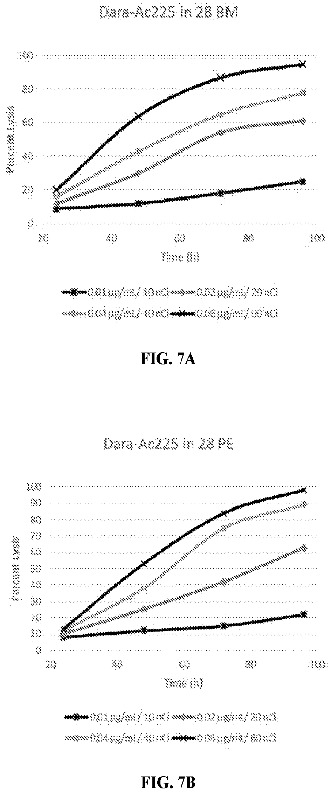

**FIG. 6** provides a summary graph of the data found in **FIGA. 5A-5D,** indicating the percent cell lysis over time for the various concentrations of ²²⁵Ac-DARA. **FIGS. 7A** and **7B** provide graphs which indicate the ability of various concentrations of ²²⁵Ac-DARA conjugates to induce lysis of 28BM and 28PE multiple myeloid cells, respectively, by ADCC over the course of 96 hours.

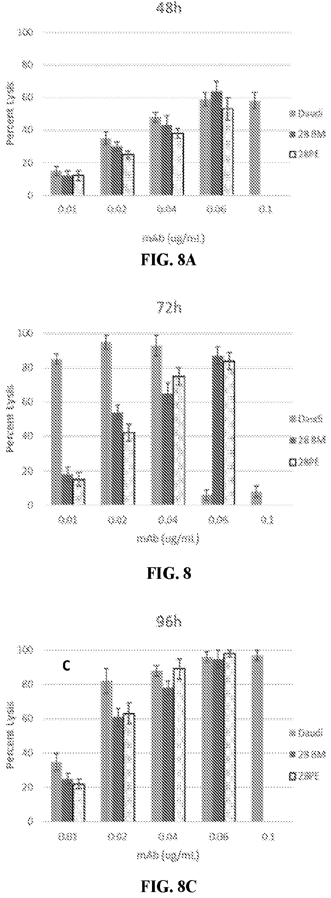

**FIGS. 8A-8C** provide bar graphs which compare cell lysis results for Daudi, 28BM, and 28PE cells on exposure to various concentration of ²²⁵Ac-DARA conjugates, wherein panels **FIGS. 8A-8C** show exposure times of 48, 72, and 96 hours, respectively.

Importantly, immunogenicity was preserved upon labeling DARA with DOTA and ²²⁵Ac. As shown in the figures, concentrations of antibodies and ²²⁵Ac-labeled constructs ranging from 0.01 µg/mL to 0.06 µg/mL were tested in each of the four cell lines. The ratio of activity : antibody utilized was 10 nCi:0.01 µg/mL. Treatment of Daudi cells with 0.01 µg/mL and 0.06 µg/mL of ²²⁵Ac-DARA resulted in 25% and 95% of cell death at 72 h, respectively, versus 5-6% cell death in the daratumumab only group. A similar time- and concentration- dependent cell death was demonstrated in the patient derived cell lines, 28PE and 28BM. No time or concentration dependent killing was observed when CD38-positive cell lines were treated with the similar activities of radiolabeled isotype-matching human control antibody IgG-²²⁵Ac, or when the negative CD38 expressing multiple myeloma cell line, U266 was treated with ²²⁵Ac-DARA.

These results demonstrate the ability to label a CD38 targeting antibody with ²²⁵Ac, wherein the high potency alpha emitter, ²²⁵Ac, improves the efficacy of DARA more than 10-fold, essentially utilizing the antibody as a vehicle while still preserving the immune functions of DARA. ²²⁵Ac is an effective payload due to its high linear energy transfer properties and short path length in tissues.

### EXAMPLE 4: Animal models

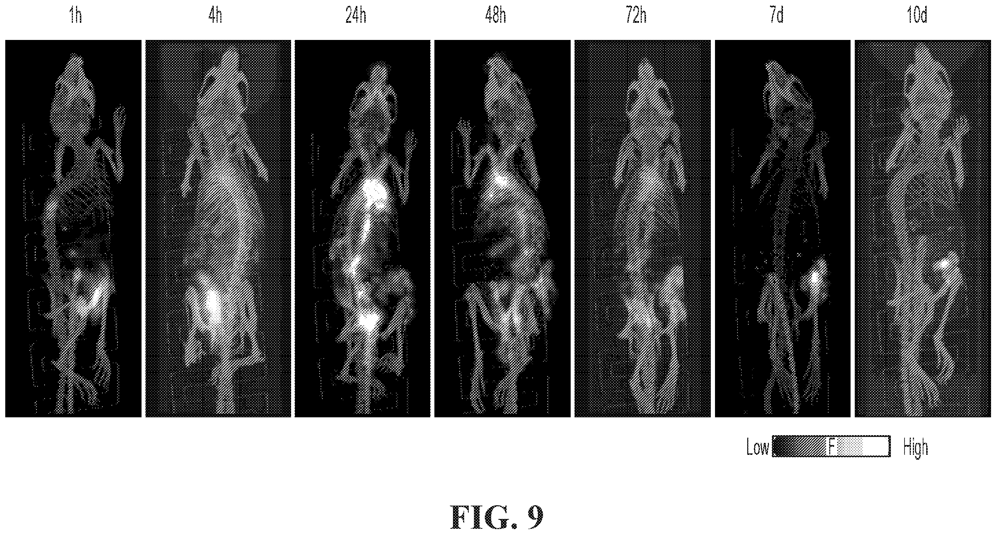

We performed microSPECT/CT imaging of ¹¹¹In-DARA in Daudi tumor-bearing mice to ascertain its specific uptake in the tumors (**FIG. 9**). For tumor induction, female SCID mice (CB17/Icr-Prkdc^{scid}/IcrIcoCrl, procured from Charles River Laboratories) were injected subcutaneously with 5x10⁶ Daudi cells in 50 µL of saline into the right flank. Tumor growth was measured with electronic calipers every 3 days, and tumor volume was calculated using the formula V=0.5(LxW²), with width being the shorter tumor diameter. On day 14 post tumor cell inoculation, when tumors reached an average volume of ~200 mm³, mice were either imaged by microSPECT/CT or treated with ¹¹¹In-DARA. For imaging, 3 mice were injected intraperitoneally (IP) with 400 µCi ¹¹¹In-DARA and imaged on a microSPECT/CT (MI Labs, Netherlands) at 1, 4, 24 hrs post-injection followed by 2, 3, 7 and 10 days.

MicroSPECT/CT imaging demonstrated noticeable localization of ¹¹¹In-DARA in tumors at 24 hrs post administration. ¹¹¹In-DARA continued to concentrate in the tumor up to 10 days, while activity had cleared from the rest of the body. **FIG. 9** shows representative images from one mouse over the entire time course.

To evaluate the side effects of the radioimmunotherapy (RIT), the tumor-bearing mice were randomized into the groups of 5 animals and injected IP with either: 100 µL saline, or unlabeled DARA at concentrations of 10 or 0.3 µg per mouse, or ²²⁵Ac-DARA at 400 nCi and 200 nCi per 0.3 µg of antibody. Mice were observed for tumor progression for 40 days and any mouse whose tumor reached 4,000 mm³ volume, or became necrotic, was humanely euthanized (**FIGS. 10A** and **10B**).

To evaluate the side effects of RIT, we performed a comprehensive safety evaluation of ²²⁵Ac-DARA by monitoring the weight of the mice (**FIG. 11A**) and assessing hematologic parameters and systemic toxicity (kidneys and liver; **FIG. 11B**). The weight of mice in the 200 nCi ²²⁵Ac-DARA group did not change throughout the experiment while mice in the 400 nCi group demonstrated only transient weight loss during the 2nd week post treatment, with rapid recovery noted in the 3rd week post-treatment. The evaluation of hematologic and toxicity parameters demonstrated that there was no statistically significant difference in any of the ²²⁵Ac-labeled versus unlabeled groups in any parameter tested. This indicated that there was no impact of the presence of ²²⁵Ac conjugate on blood parameters or evidence of increased liver damage as measured by aspartate aminotransferase (AST) and alanine transaminase (ALT) levels in treated mice. Further, there was no change in creatinine and blood urea nitrogen (BUN) demonstrating the absence of any apparent kidney toxicity.

## Claims

1. A pharmaceutical composition comprising a DOTA-conjugated daratumumab for use in a method of treating a disease or disorder involving cells expressing CD38, the method comprising:
administering to a subject an effective amount of said pharmaceutical composition, wherein
the DOTA-conjugated daratumumab consists of an actinium ²²⁵Ac-labeled portion,
and an unlabeled portion,
wherein the cells expressing CD38 are CD38-expressing cancer cells, solid tumor cells, hematological malignancy cells, or CD38-expressing T-cells, B-cells, NK cells, or plasma cells.

2. The pharmaceutical composition for use of claim 1, wherein the hematological malignancy cells comprise multiple myeloma cells, acute lymphocytic leukemia cells, acute myeloid leukemia cells, chronic lymphocytic leukemia cells, chronic myeloid leukemia cells, Hodgkin's lymphoma cells, non-Hodgkin's lymphoma cells, T-LGL leukemia cells, NK cell leukemia cells, or hairy cell leukemia cells.

3. The pharmaceutical composition for use of claim 1 or 2, wherein the monoclonal antibody consists of 5 to 50 wt.% of the ²²⁵AC labeled portion and 50 to 95 wt.% of the unlabeled portion.

4. The pharmaceutical composition for use of claim 1, wherein the effective amount comprises a protein dose of 1ug/kg to 1mg/kg antibody and a radiation dose of 0.1uCi/kg to 5uCi/kg.

5. The pharmaceutical composition for use of claims 1 to 4, wherein the effective amount is administered as a single dose.

6. The pharmaceutical composition for use of claims 1 to 5, wherein the effective amount is administered as two equal fractionated doses, wherein a second dose is administered 3 to 8 days after a first dose.

7. The pharmaceutical composition for use of claims 1 to 6, further comprising: administering one or more further therapeutic agents before or after administration of the pharmaceutical composition comprising a DOTA-conjugated daratumumab.

8. The pharmaceutical composition for use of claim 7, wherein the one or more further therapeutic agents comprises a chemotherapeutic agent, an anti-inflammatory agent, an immunosuppressive, an immunomodulatory agent, or a combination thereof.

9. The pharmaceutical composition for use of claim 7, wherein the one or more further therapeutic agents comprises an antimyeloma agent.

10. The pharmaceutical composition for use of claim 9, wherein the antimyeloma agent is selected from dexamethasone, melphalan, doxorubicin, bortezomib, lenalidomide, prednisone, carmustine, etoposide, cisplatin, vincristine, cyclophosphamide, and thalidomide.

11. An article of manufacture comprising
(a) a pharmaceutical composition comprising:
daratumumab, wherein 5 to 50 wt.% the daratumumab is a DOTA-conjugated daratumumab labeled with actinium ²²⁵Ac;
and 50 to 95 wt.% of the daratumumab is unlabeled; and a pharmaceutically acceptable carrier; and
(b) a label instructing a user to administer to a subject an amount of the antibody effective to treat a disease or disorder involving cells expressing CD38.

12. The article of manufacture of claim 11, wherein the pharmaceutical composition comprises a protein dose of 1ug/kg to 1mg/kg antibody and a radiation dose of 0.1 uCi/kg to 5uCi/kg.

## Patentansprüche

1. Pharmazeutische Zusammensetzung, umfassend ein DOTA-konjugiertes Daratumumab zur Verwendung in einem Verfahren zum Behandeln einer Krankheit oder Störung, an der CD38-exprimierende Zellen beteiligt sind, das Verfahren umfassend:
Verabreichen einer wirksamen Menge der pharmazeutischen Zusammensetzung an einen Patienten, wobei das DOTA-konjugierte Daratumumab aus einem mit Actinium ²²⁵Ac markierten Anteil und einem unmarkierten Anteil besteht,
wobei die CD38-exprimierenden Zellen CD38-exprimierende Krebszellen, solide Tumorzellen, hämatologische Malignomzellen oder CD38-exprimierende T-Zellen, B-Zellen, NK-Zellen oder Plasmazellen sind.

2. Pharmazeutische Zusammensetzung zur Verwendung nach Anspruch 1, wobei die hämatologischen Malignomzellen multiple Myelomzellen, akute lymphatische Leukämiezellen, akute myeloische Leukämiezellen, chronische lymphatische Leukämiezellen, chronische myeloische Leukämiezellen, Hodgkin-Lymphom-Zellen, Non-Hodgkin-Lymphom-Zellen, T-LGL-Leukämiezellen, NK-Zell-Leukämiezellen oder Haarzell-Leukämiezellen umfassen.

3. Pharmazeutische Zusammensetzung zur Verwendung nach Anspruch 1 oder 2, wobei der monoklonale Antikörper aus 5 bis 50 Gew.-% des ²²⁵AC markierten Anteils und 50 bis 95 Gew.-% des unmarkierten Anteils besteht.

4. Pharmazeutische Zusammensetzung zur Verwendung nach Anspruch 1, wobei die wirksame Menge eine Proteindosis von 1 ug/kg bis 1 mg/kg Antikörper und eine Strahlungsdosis von 0,1 uCi/kg bis 5 uCi/kg umfasst.

5. Pharmazeutische Zusammensetzung zur Verwendung nach den Ansprüchen 1 bis 4, wobei die wirksame Menge als Einzeldosis verabreicht wird.

6. Pharmazeutische Zusammensetzung zur Verwendung nach den Ansprüchen 1 bis 5, wobei die wirksame Menge als zwei gleiche fraktionierte Dosen verabreicht wird, wobei eine zweite Dosis 3 bis 8 Tage nach einer ersten Dosis verabreicht wird.

7. Pharmazeutische Zusammensetzung zur Verwendung nach den Ansprüchen 1 bis 6, ferner umfassend: Verabreichen eines oder mehrerer weiterer therapeutischer Wirkstoffe vor oder nach Verabreichung der pharmazeutischen Zusammensetzung, die ein DOTA-konjugiertes Daratumumab umfasst.

8. Pharmazeutische Zusammensetzung zur Verwendung nach Anspruch 7, wobei der eine oder die weiteren therapeutischen Wirkstoffe ein Chemotherapeutikum, einen entzündungshemmenden Wirkstoff, ein Immunsuppressivum, einen immunmodulatorischen Wirkstoff oder eine Kombination davon umfassen.

9. Pharmazeutische Zusammensetzung zur Verwendung nach Anspruch 7, wobei der eine oder die weiteren therapeutischen Wirkstoffe einen Anti-Myelom-Wirkstoff umfassen.

10. Pharmazeutische Zusammensetzung zur Verwendung nach Anspruch 9, wobei der Anti-Myelom-Wirkstoff ausgewählt ist aus Dexamethason, Melphalan, Doxorubicin, Bortezomib, Lenalidomid, Prednison, Carmustin, Etoposid, Cisplatin, Vincristin, Cyclophosphamid und Thalidomid.

11. Ein Herstellungserzeugnis, umfassend
(a) eine pharmazeutische Zusammensetzung, umfassend:
Daratumumab, wobei 5 bis 50 Gew.-% des Daratumumabs ein mit Actinium ²²⁵Ac markiertes DOTA-konjugiertes Daratumumab sind;
und 50 bis 95 Gew.-% des Daratumumabs unmarkiert sind; und
einen pharmazeutisch verträglichen Träger; und
(b) eine Kennzeichnung, die den Anwender anweist, einem Patienten eine zur Behandlung einer Erkrankung oder Störung, an der CD38-exprimierende Zellen beteiligt sind, wirksame Menge des Antikörpers zu verabreichen.

12. Herstellungserzeugnis nach Anspruch 11, wobei die pharmazeutische Zusammensetzung eine Proteindosis von 1 ug/kg bis 1 mg/kg Antikörper und eine Strahlungsdosis von 0,1 uCi/kg bis 5 uCi/kg umfasst.

## Revendications

1. Composition pharmaceutique comprenant un daratumumab conjugué au DOTA pour utilisation dans un procédé de traitement d'une maladie ou d'un trouble impliquant des cellules exprimant CD38, le procédé comprenant :
l'administration à un sujet d'une quantité efficace de ladite composition pharmaceutique,
le daratumumab conjugué au DOTA consistant en une portion marquée par ²²⁵Ac et une portion non marquée,
les cellules exprimant CD38 étant des cellules cancéreuses exprimant CD38, des cellules tumorales solides, des cellules hématologiques malignes ou des cellules T, des cellules B, des cellules NK ou des cellules plasmatiques exprimant CD38.

2. Composition pharmaceutique pour utilisation selon la revendication 1, dans laquelle les cellules malignes hématologiques comprennent des cellules de myélome multiple, des cellules de leucémie lymphocytaire aiguë, des cellules de leucémie myéloïde aiguë, des cellules de leucémie lymphocytaire chronique, des cellules de leucémie myéloïde chronique, des cellules de lymphome de Hodgkin, des cellules de lymphome non hodgkinien, des cellules de leucémie T-LGL, des cellules de leucémie à cellules NK ou des cellules de leucémie à cellules ciliées.

3. Composition pharmaceutique pour utilisation selon la revendication 1 ou 2, dans laquelle l'anticorps monoclonal consiste en 5 à 50 % en poids de la portion marquée par ²²⁵Ac et 50 à 95 % en poids de la portion non marquée.

4. Composition pharmaceutique pour utilisation selon la revendication 1, dans laquelle la quantité efficace comprend une dose de protéine de 1 ug/kg à 1 mg/kg d'anticorps et une dose de rayonnement de 0,1 uCi/kg à 5 uCi/kg.

5. Composition pharmaceutique pour utilisation selon les revendications 1 à 4, dans laquelle la quantité efficace est administrée en une seule dose.

6. Composition pharmaceutique pour utilisation selon les revendications 1 à 5, dans laquelle la quantité efficace est administrée en deux doses fractionnées égales, la deuxième dose étant administrée 3 à 8 jours après une première dose.

7. Composition pharmaceutique pour utilisation selon les revendications 1 à 6, comprenant en outre :
l'administration d'un ou plusieurs autres agents thérapeutiques avant ou après l'administration de la composition pharmaceutique comprenant un daratumumab conjugué au DOTA.

8. Composition pharmaceutique pour utilisation selon la revendication 7, dans laquelle les un ou plusieurs autres agents thérapeutiques comprennent un agent chimiothérapeutique, un agent anti-inflammatoire, un immunosuppresseur, un agent immunomodulateur, ou une combinaison de ceux-ci.

9. Composition pharmaceutique pour utilisation selon la revendication 7, dans laquelle les un ou plusieurs autres agents thérapeutiques comprennent un agent antimyélomateux.

10. Composition pharmaceutique pour utilisation selon la revendication 9, dans laquelle l'agent antimyélomateux est choisi parmi la dexaméthasone, le melphalan, la doxorubicine, le bortezomib, le lénalidomide, la prednisone, la carmustine, l'étoposide, le cisplatine, la vincristine, le cyclophosphamide et la thalidomide.

11. Article de fabrication comprenant
(a) une composition pharmaceutique comprenant :
du daratumumab, 5 à 50 % en poids du daratumumab étant un daratumumab conjugué au DOTA marqué par l'actinium ²²⁵Ac; et 50 à 95 % en poids du daratumumab n'étant pas marqué ; et
un support pharmaceutiquement acceptable ; et
(b) une étiquette indiquant à l'utilisateur d'administrer à un sujet une quantité de l'anticorps efficace pour traiter une maladie ou un trouble impliquant des cellules exprimant CD38.

12. Article de fabrication selon la revendication 11, dans lequel la composition pharmaceutique comprend une dose de protéine de 1 ug/kg à 1 mg/kg d'anticorps et une dose de rayonnement de 0,1 uCi/kg à 5 uCi/kg.
